# EUROPEAN PATENT SPECIFICATION

(11) **EP 3 223 898 B1**
(45) Date of publication and mention of the grant of the patent: **27.05.2020**
(21) Application number: 14906746.4
(22) Date of filing: 26.11.2014
(51) Int. Cl.: A61M 29/02, A61F 5/00

(54) **ANCHORABLE SIZE-VARYING GASTRIC BALLOONS FOR WEIGHT LOSS**
VERANKERBARE GRÖSSENVARIABLE GASTRISCHE BALLONS FÜR GEWICHTSVERLUST
BALLONNETS GASTRIQUES À TAILLE VARIABLE ANCRABLES POUR PERTE DE POIDS

(43) Date of publication of application: 04.10.2017
(73) Proprietor: EZ-OFF Weightloss, LLC, Leawood, KS 66211 (US)
(72) Inventor: PATTISON, Mary, Kansas City, Missouri 64112 (US); PATTISON, Charles Phillip, Kansas City, Missouri 64112 (US); LOWRY, Stephen J., Kansas City, Missouri 64112 (US); MOLOS, Mark, Kansas City, Missouri 64112 (US)
(74) Representative: Hannke, Christian
(86) International application number: PCT/US2014/067697
(87) International publication number: WO 2016/085503

(56) References cited:
- WO-A1-2013/023675
- WO-A1-2014/145799
- US-A- 5 259 399
- US-A- 5 993 473
- US-A1- 2002 055 757
- US-A1- 2003 158 569
- US-A1- 2004 059 289
- US-A1- 2005 240 279
- US-A1- 2006 025 799
- US-A1- 2009 204 067
- US-A1- 2010 152 764
- US-A1- 2010 152 764
- US-A1- 2010 249 822
- US-A1- 2010 312 047
- US-A1- 2012 095 495

## Description

### BACKGROUND OF INVENTION

Provided herein are systems and methods related to obesity treatment and weight loss by a gastric restriction device, and that is implemented at least in part by transabdominal access to the gastric environment. The gastric restriction device corresponds to a size-varying gastric balloon that can be inflated to a deployed volume that occupies a significant fraction of the stomach volume and that is reliably anchored so as to prevent unwanted balloon migration.

In recent years obesity and related disorders, such as diabetes and atherosclerotic cardiovascular disease, have increased substantially. When compliance with diet, exercise and behavioral therapy fail to achieve weight loss, pharmacotherapy may be instituted. However, pharmacotherapy has had only modest success and may be discontinued if a patient experiences unpleasant side-effects. Long term safety of pharmaceutical use for treatment of obesity is uncertain, and patients generally regain lost weight when the therapy is discontinued. A variety of surgical treatments have recently become available for obesity, but typically as a last resort. Those surgical treatments have the advantage of more rapid initial weight loss and remission of diabetes mellitus than other non-invasive therapies. However, surgery is expensive, subject to risks of morbidity and mortality, and its efficacy may be reduced by patient noncompliance with post-surgical dietary restrictions. If patients fail to limit food intake, their bodies may undergo compensatory anatomical changes that partially overcome the effects of surgery. The most invasive surgical procedures tend to achieve the greatest long term percent change in weight, but also tend to be the most costly, require longer periods of recuperation and careful long term management of nutrients to avoid malnutrition.

Although there have been various attempts to treat obesity by minimally invasive procedures, those attempts still suffer from significant disadvantages including one or more of being relatively complex, difficult to modify or remove, result in permanent anatomical changes, or undergo efficacy degradation with time. The systems and related methods provided herein address these limitations in that the gastric-occupying devices are robust and long-lasting, while being easily inserted, positioned, manipulated and removed from a patient using a minimally invasive procedure.

### SUMMARY OF THE INVENTION

A common problem with gastric balloons that are inflated to function as a gastric restriction device is that they can be difficult to control and maintain in an optimum position within the stomach. During use and over extended periods of time, the balloons tend to migrate and may increase the risk of malfunction, gastric obstruction or the like, and patient discomfort requiring active intervention and potentially balloon removal. Those drawbacks are a reflection of the difficulty in reliably anchoring the balloon to the stomach wall. One reason for this difficulty is that it is a challenge to handle and manipulate the balloon within the stomach lumen and reliably anchor the balloon to the stomach in a manner that does not adversely impact balloon function and the gastric environment.

Endoscopic procedures are limited in that the devices must be able to be inserted through the esophagus without causing undue irritation. This, therefore, inherently constrains the number and size of devices that can employed. Furthermore, it can be difficult to reliably control and position multiple endoscopes to specific locations. For these reasons, endoscopic procedures tend to be relatively simple with one endoscope and confined to the upper GI tract, generally the esophagus, stomach and duodenum so as to avoid increasing risk of complications. Such a device that is implanted in the stomach by means of percutaneous endoscopic gastrostomy (PEG) through the esophagus is described for example in US 2010/152764 A1.

Because of the inherent limitations of endoscopic procedures, laparoscopic procedures are also used for introduction of a medical instrument through the abdominal wall, thereby accessing the body's organs in an extra-luminal manner. In contrast to an endoscopic procedure where an instrument is introduced through the mouth, laparoscopy requires an opening be made through the abdominal wall for medical instrument access to the inside of the patient, such as the intraperitoneal space. This is typically achieved by using a trocar or other instrument having a sharp distal tip and a passage to provide a working passage for a medical instrument. Those trocars, however, suffer from inherent disadvantages and associated risks. First, the peritoneal membrane must be actively punctured from outside the patient to provide instrument access. This can result in increased risk of infection or other complications. Second, it can be difficult to reliably secure the trocar or other cannula-type element, including for extended periods of time with attendant movements on the trocar from instrument use during the surgical procedure. Lack of a safe, stable, and reliable working channel that traverses the abdominal wall can lead to unwanted complications. Furthermore, current technology requires that if a laparoscopic physician wishes to place a trocar into the gastric lumen via standard laparoscopic technique, they must make multiple abdominal incisions which will allow them to grasp the external wall of the stomach and then fix it to the abdominal wall by multiple suture/staple or other fixation methods. Once the stomach has been fixed in a safe and stable manner, the physician must make an external full thickness incision through the gastric wall and then externally place a surgical trocar that can be used for both passage and manipulation of laparoscopic instruments into the gastric lumen. The trocar must be air-tight to allow air insufflation of the stomach for both internal vision as well as manipulation of instruments. Trocars usually have internal air-tight seals for passage of instruments. The surgeon, however, must ensure an air-tight seal around the external aspect of the trocar to prevent air leakage on an external surface where the trocar contacts the gastric wall. This procedure is complex, with an attendant risk to establish intra-gastric access via laparoscopic surgery, and reflects the inherent difficulty in trocar insertion into a non-solid organ, such as a stomach. The problem of gastric lumen access for balloon manipulation and handling is addressed herein by specially configured trans-abdominal gastric systems having a cannula that provides a reliable and robust working channel to the gastric lumen. The working channel is easily achieved without any of the above-reference drawbacks. The working channel, or multiple working channels as desired, provides the ability to reliably and readily triangulate medical instruments to for superior control within the gastric lumen. In combination with a size-varying gastric balloon and attendant balloon anchors, precise and reliable balloon placement within the gastric lumen is achieved, in a non-permanent and non-surgical manner. For example, the balloon may be anchored to the gastric wall via one or more balloon anchors. This avoids direct suturing or stapling of the balloon to the gastric wall. If the balloon is to be removed from the patient, the anchors are simply released from the gastric wall, thereby avoiding the complications associated with cutting of sutures or removal of staples which directly attach the balloon to the gastric wall. Accordingly, the systems and methods provided herein avoid direct connections between the balloon to the gastric wall without sacrifice of reliable and long-term balloon positioning that minimizes the risk of gastric obstruction.

Provided are systems and related methods for obesity treatment. In particular, size-varying balloons positioned in the stomach lumen are inflated so as to occupy a substantial fraction of the stomach lumen. The balloons are configured to avoid unwanted migration during use by the use of specially configured anchors and attendant balloon and tissue manipulation through a transabdominal gastric cannula. In addition, the cannula may be used to deliver a fluid (e.g., liquid or air) from a location outside the patient to inside the balloon to expand its volume, such as via a fluid conduit positioned through the cannula lumen. Alternatively, a fluid port may be endoscopically accessed for introduction/removal of fluid to control balloon volume and, therefore, the fraction of the stomach lumen occupied by the balloon. The balloon itself may be specially shaped to mimic sleeve gastrectomy without permanent surgical resection. In fact, the systems described herein are readily controllable and removable, thereby avoiding adverse impacts should a subsequent surgical procedure arise.

Specific advantages of the systems and methods of the present invention include, adjustable balloon size permits the ability to tailor the procedure to the individual patient. In addition, the unique shape of certain balloons that mimic sleeve gastrectomy achieves the benefit of surgical sleeve gastrectomy without the disadvantages associate with permanent surgical resection. The anchors of the present invention avoid more permanent type fastening means that are more difficult to reverse without impacting the ability to prevent balloon migration. The systems and procedures are less invasive and are not associated with a permanent anatomic change. Furthermore, general endotracheal anesthesia (GETA) is avoided, and there is decreased mortality and risk of malnutrition.

In an embodiment, the invention is a gastric balloon system for treatment of obesity in a patient. The system may be broadly described in terms of certain general components. One important component is a size-varying gastric balloon, whose function is to provide a feeling of fullness or satiety to a patient, thereby decreasing appetite. Particularly useful size-varying gastric balloons are those having an annulus and elements to avoid distal migration after deployment in the stomach lumen, such as one or more gastric anchor configured to connect the balloon to the stomach wall, where the anchors still, however, permit constrained movement. Another important component, in certain aspects, is a transabdominal gastric cannula, also referred herein as a transabdominal gastric system. Examples of various such gastric cannula systems are provided herein, and also in, for example, U.S. Pat. App. 14/451,108 titled "Transabdominal Gastric Surgery System and Method" filed Aug. 4, 2014, and 14/554,337 titled "Transabdominal Gastric Device and Method" filed Nov. 26, 2014. The transabdominal gastric cannula provides certain functions, including as a reliable anchor for one or more of balloon insertion, manipulation, inflation, deflation and/or attachment. Furthermore, additional transabdominal gastric cannula may be used to provide added flexibility with respect to balloon handling. Finally, a malabsorptive sleeve may be used in combination with a gastric balloon and/or a transabdominal gastric cannula. Each of those three components is independently selectable and compatible with the other components, so that any balloon may be used with any transabdominal gastric cannula and/or with any malabsorption sleeve.

In an embodiment the gastric balloon system for treatment of obesity comprises a transabdominal gastric cannula for gastric balloon insertion and/or manipulation in a stomach lumen and a gastric balloon. The cannula is a passage that, during use, provides access to the gastric environment by a working channel. Accordingly, the working channel is an important aspect in that improved balloon manipulation, such as for balloon anchoring to a gastric wall is achieved. The gastric cannula may comprise an outer end, an inner end, and a central portion having an outer-facing surface that extends between the inner end and the outer end, and an inner-facing surface that defines a lumen configured to receive a portion of a medical instrument that traverses between the outer end and the inner end. An internal anchor is connected to the inner end and has a surface shape configured to secure the system against an interior surface of a gastric wall or peritoneal surface. In this aspect, connected is used broadly to include a single piece of material having a portion defined as an internal anchor from which a cannula extends. Alternatively, the connection may refer to two separate pieces, the cannula and internal anchor, which are permanently or removably connected. An external anchor is removably and translationally connected to the cannula outer-facing surface and has a surface shape configured to secure the system against a skin surface.an outer end. A size-varying gastric balloon is configured for delivery or insertion to a stomach lumen through the transabdominal gastric cannula or endoscopically via the patient's esophagus. The gastric balloon may comprise a gastric balloon surface; an internal volume defined by the gastric balloon surface; a gastric balloon anchor connected to the gastric balloon surface configured to reliably position and anchor the size-varying gastric balloon in a gastric environment. For control of the internal volume of the size-varying gastric balloon, a fluid conduit or port may be fluidically connected thereto. The fluid conduit or port may have a first fluid end and a second fluid end, wherein the first fluid end is fluidically connected to the size-varying gastric balloon internal volume and the second fluid end is positioned externally or internally relative to the patient for connection to a fluid source. The fluid conduit is configured to introduce a fluid to gastric balloon internal volume to increase the internal volume to a deployed internal volume and provide a gastric balloon deployed configuration. The second end of the fluid conduit may be positioned outside the patient for a convenient access point for a fluid source or sink for balloon inflation or deflation, respectively, or via a subcutaneous port. Alternatively, the second end of the fluid conduit may be positioned internally and a fluid source introduced endoscopically, such as via a needle and port through the balloon wall. The fluid, as desired, may be a gas, a liquid, or a viscoelastic material that flows under an applied force, such as a gel.

An important aspect of the instant invention is the ability to precisely position and maintain the gastric balloon in the stomach lumen. This reduces risk of balloon migration and attendant gastric obstruction and patient discomfort. In an aspect, the gastric balloon anchor is connected to the transabdominal gastric cannula, thereby tethering the gastric balloon in a desired position within the stomach lumen. Alternatively, or in addition thereto, the balloon is anchored independently to the stomach wall by one or more anchors connected to the balloon outer surface.

Accordingly, in another aspect the invention is a gastric balloon anchor, such as an anchor configured for use with any type of size-varying gastric balloon. The gastric balloon anchor may be used to retro-fit a conventional size-varying gastric balloon, so that any balloon known in the art may be used with the gastric cannulas provided herein for reliable anchoring, thereby facilitating long-term balloon insertion without unwanted side effects. The anchor may be further described as providing constrained movement or motion. "Constrained motion" refers to the anchor that provides some degree of movement relative to the balloon and the gastric wall. In an aspect, constrained motion refers to an at least partial rotational motion movement in the anchor. Such constrained motion can accommodate various forces and motions in the stomach and movement of the stomach wall. This provides a functional benefit of prolonging balloon lifetime and avoiding patient discomfort commonly associated with conventional gastric balloons.

In an aspect, the anchor comprises an anchor mount having an opening for receiving one or more pre-sutures or preclosure sutures. A fastener has a first end that is connected to the gastric balloon surface and a second end connected to the anchor mount. The connection may be a movable connection, so that the anchor mount can move relative to the fastener second end to the sutures or corresponding stomach wall after suture closure.

The anchor mount may be a ring that receives a second end that is a fastener coupling, such as a fastener ring. In this manner, the two interlocking rings may move relative to each other, but in a manner that is constrained, thereby ensuring unwanted balloon migration is avoided while also avoiding undesired effects associated with a more rigid anchor connection.

The fastener first end may be embedded in the size-varying gastric balloon wall, such as during balloon manufacture. Alternatively, an already constructed size-varying gastric balloon may be retrofitted with the anchor, such as by affixing the fastener first end to the balloon outer surface, such as by an adhesive.

In an aspect, any of the gastric balloon systems further comprise any of the gastric balloon anchors provided herein.

In an aspect, the fluid conduit is connected to an anterior portion of the size-varying gastric balloon and traverses the cannula lumen. In this aspect, therefore, the fluid cannula may serve the dual functions of being an anchor (such via the transabdominal gastric cannula) and to provide balloon inflatability/deflatability.

In another embodiment, the fluid conduit is connected to a cephalad portion of the size-varying gastric balloon and traverses a patient's esophagus. "Cephalad portion" refers to the portion of the balloon that is oriented toward the top of the stomach and accessible by the esophagus.

Any of the systems and methods describe may have a second gastric cannula, such as to provide independent access to a medical device, implant, or the like. In an aspect, the system further comprises a second fluid conduit and a second transabdominal gastric cannula through which each fluid conduit independently traverses, each first fluid conduit end connected to an anterior portion of the size-varying gastric balloon with the connections separated from each other by a fluid conduit separation distance that is greater than or equal to 15 cm to provide a two inflation ports, deflation and inflation ports, or two deflation ports. Use of two gastric cannulas provides the ability of instrument triangulation and further improved control and manipulation of the balloon, anchor and stomach wall tissue. In this manner, fundamentally improved balloon anchoring is achieved.

The configuration of the transabdominal gastric cannula provides the ability for reliable removal, so that after activation of a gastric balloon deployed configuration, the transabdominal gastric cannula may be removed from the patient, including providing reliable balloon anchoring and tissue defect closure in a single, convenient and reliable step. Alternatively, the system may be configured for long-term use of the transabdominal gastric cannula. For example, for a transabdominal gastric cannula having a low profile, and after deployment to the gastric balloon deployed configuration, the transabdominal gastric cannula may be retained in the patient.

The fluid conduit may be configured to remove a fluid from the internal volume to decrease the gastric balloon internal volume, such as from a deployed state to a relaxed state, such as ahead of balloon removal or for a controlled decrease in the internal volume, such as by at least 10%, 20%, 50% or 90%.

The fluid conduit may be connected to a fluid source external to the patient for introducing a fluid to the internal volume to control the size-varying gastric balloon shape and size. The fluid may be a gas, a liquid or a gel. For a balloon having air, the gas source may simply be the surrounding environment. For a liquid the liquid may be water or saline, for example.

Any of the systems provided herein may have a fluid conduit connected to the transabdominal gastric cannula. Alternatively or in addition thereto, a fluid conduit that is positioned within the gastric lumen may be accessed by an endoscopically introduced fluid source and/or a laparoscopically introduced fluid source through the gastric cannula working channel.

In an embodiment, provided is a size-varying gastric balloon having an annulus in a deployed configuration configured to align in the stomach to pass food and liquid in a direction from an esophageal exit toward a pyloric sphincter. The annulus facilitates movement of materials naturally through the stomach and minimizes risk of a gastric obstruction, without unduly sacrificing the feeling of fullness by the patient. This further enhances deployed balloon stability, longevity, and decrease in complications associated with other balloons. Furthermore, the transabdominal gastric cannula provides an important functional benefit of enhanced handling in the stomach lumen and attendant improvement in balloon anchoring and positioning.

The annulus may have an average diameter that is greater than or equal to 0.5 cm and less than or equal to 5 cm and a deployed volume that occupies at least 75% of the stomach lumen. "Average diameter" may be determined in any number of ways, and is a reflection that given the flexible nature of the balloon and curved nature of the annulus, the diameter may not be constant. For example, a diameter may be obtained for a select number of points along the annulus, and an attendant average calculated from those points. The annulus may be further described in terms of a length, such as a length that is greater than or equal to 5 cm and less than or equal to 50 cm.

The annulus may be a straight line passage or may be curved. During use the annulus has an entry end configured to be aligned and separated from a patient's esophageal exit; and an exit end configured to be aligned and separated from a patient's pyloric sphincter. In this context, "alignment" refers to the ability of a material to pass from the esophagus into the annulus inlet or from out of the annulus exit and out the stomach via the pyloric sphincter, without substantial risk of blockage. The outermost edge of the balloon may be separated from the inner surface of the gastric balloon, but at a sufficiently low value to minimize or avoid food being trapped between the balloon and the stomach wall.

The gastric balloon system of the instant invention may be described as having, during use, at least 75% of the stomach lumen occupied by a deployed balloon and less than 25% of the stomach lumen unoccupied by the deployed balloon, wherein at least 60% of the unoccupied stomach lumen volume is in a distal stomach portion adjacent to a pyloric sphincter to avoid gastric obstruction; and less than 20% of the unoccupied stomach lumen is in a proximal stomach portion adjacent to an esophageal sphincter. In another aspect, the annulus has an entry end that is separated from the esophageal exit by an esophageal distance that is between 2 cm and 5 cm; and said annulus exit end is separated from the pyloric sphincter by a pyloric distance that is between 2 cm and 5 cm. In this manner, the likelihood of a gastric obstruction is minimized or avoided. The unique construction of the balloon and balloon anchors ensures that the balloon does not migrate in a distal direction during use, thereby maintaining the large unoccupied distal stomach portion relative to the proximal stomach portion.

In an embodiment, during use the entry end and the exit end are substantially fixably positioned within a gastric environment by a plurality of gastric balloon anchors. In this context, "substantially fixably positioned" refers to minimal migration of the ends in a distal direction, such as less than 1 cm or less than 5 mm over the course of the treatment. The systems and methods are designed for a treatment course of greater than 1 month, greater than 3 months, greater than 6 months, or between about 1 month and three years. The specially-configured anchors that connect the balloon to the gastric wall and accessed by the working channel(s) provides some movement, described herein as constrained motion that allows much longer balloon implant function compared to conventional systems.

The gastric balloon anchors may each comprise a first end operably connected to the gastric balloon surface and a second end configured to connect to a gastric wall during use. Alternatively, the anchor can connect to the transabdominal gastric cannula, such as via a tether-type mechanism. This aspect is particularly useful for those applications where the gastric cannula remains in the patient long-term. Any of the gastric balloon anchors may have one end that is integrally inserted into the balloon wall. Alternatively, the end may be affixed to the outer-surface of the balloon, such as with an adhesive. In this manner, any conventional gastric balloon known in the art may be retro-fitted with an anchor for use with any of the systems or methods provided herein.

An example of another type of gastric balloon anchor is the fluid conduit connected to the internal volume that corresponds to a percutaneous catheter that passes through and is connected to the transabdominal gastric cannula.

Any of the systems and methods provided herein may further comprise an elongated sleeve configured for insertion into a portion of a patient's small intestine, the elongated sleeve having a lumen and a proximal end connected thereto, the proximal end configured for positioning at or adjacent to a pyloric sphincter and aligned with the gastric balloon annulus during use.

In an embodiment, the invention is a gastric balloon system for treatment of obesity comprising a size varying gastric balloon having: an outer-facing surface; an inner-facing surface, wherein the inner-facing surface defines an annulus in a deployed configuration, said annulus having an entry, an exit, an annulus length between the entry and exit that is greater than or equal to 5 cm and less than or equal to 50 cm; and an annulus diameter that is greater than or equal to 0.5 cm and less than or equal to 5 cm; an interior volume defined by the outer-facing surface and the inner-facing surface; a fluid port or cannula having a first end fluidically connected to the interior volume for controllably introducing a fluid to increase a volume of the size-varying gastric balloon. Such a balloon geometry advantageously avoids gastric obstruction and can optionally be used with any of a wide variety of transabdominal gastric cannula.

One example is a transabdominal gastric cannula having a lumen or working channel along which the percutaneous cannula traverses and that positionably secures the size-varying gastric balloon in a stomach lumen. The transabdominal gastric cannula may further comprise an internal anchor configured to secure said transabdominal gastric cannula to a gastric surface; and an external anchor configured to contact a skin surface. The internal and external anchors reliably anchor the transabdominal gastric cannula to the gastric surface during use. In an aspect, the fluid port may be a percutaneous cannula that traverses the working channel to provide the ability to inflate/deflate the gastric balloon in the stomach lumen.

As desired, a second transabdominal gastric cannula for introducing one or more instruments to the gastric environment may be used, such as an instrument to assist with balloon and anchor manipulation and/or insertion of a malabsorption sleeve in the small intestine.

The gastric balloon system may have one or more gastric balloon anchors, each having a first end connected to the outer facing balloon surface, and a second end configured to connect to a gastric surface. The anchors can be specially configured to provide constrained balloon motion and movement, thereby increasing balloon lifetime with attendant improved patient outcome with respect to weight loss without substantial side effects. In a gastric balloon deployed configuration, the gastric balloon and annulus each may be curved in a longitudinal direction with a substantially cylindrical outer shape to substantially align the longitudinal direction with a stomach lumen during use; and at least 75%, at least 80%, or at least 90% of the stomach lumen is occupied by the gastric balloon. One advantage of the size-varying balloon, is the ability to adjust balloon volume depending on individual patient needs or responsiveness.

Also provided are various methods for treating obesity by using any of the systems described herein. In an embodiment, the method of treating obesity in a patient is by inserting a size-varying gastric balloon in an undeployed configuration into a stomach lumen, wherein the size-varying gastric balloon optionally has an annulus; securing the size-varying gastric balloon to a stomach wall or a transabdominal gastric cannula, such as via one or more gastric anchors, wherein the optional annulus is positioned in a longitudinal orientation relative to the stomach lumen; and introducing a fluid to an internal volume of the size-varying gastric balloon to provide the size-varying gastric balloon in a deployed configuration that occupies at least 75% of the stomach lumen, wherein the annulus is substantially aligned with the esophageal and pyloric sphincters to reduce risk of a stomach blockage during use.

The deployed configuration may provide a balloon-pyloric sphincter open space volume that is greater than a balloon-esophageal sphincter open space volume throughout a treatment window. This aspect is important to minimize the risk of a gastric block and/or patient discomfort associated with food-material build-up in the pyloric sphincter region by ensuring sufficient and maintained separation between the balloon distal end and the pyloric sphincter.

In an aspect, the inserting step comprises inserting a transabdominal gastric surgical system through an abdominal wall by a retrograde introduction, the inserted transabdominal gastric surgical system having a cannula lumen with an inner end connected to an internal anchor and an outer end connected to an external anchor, with a patient's abdominal wall positioned therebetween. The gastric balloon introduced from outside the patient may be endoscopically introduced, or introduced via the working channel, along the cannula lumen, and into the stomach lumen. Optionally, the gastric balloon is anchored to the transabdominal gastric system, such as at the internal anchor with a tether and/or via a fluid conduit that is fluidically connected to balloon and traverses the cannula lumen and through an instrument port or that is connected to a pressure port.

A second transabdominal gastric surgical system may be inserted through the abdominal wall at a different location than the first through which the balloon is inserted. The introduced gastric balloon may be manipulated with a medical instrument that traverses the second transabdominal gastric surgical system. The manipulation may refer to placement of the balloon with a grasper and/or fastening of a balloon anchor to the gastric wall. Multiple working channels from multiple gastric cannula provide a convenient platform to triangulate instruments at a desired anchor point, including by visualization with an endoscope.

In an aspect, the securing step comprises attaching one or more gastric balloon anchors to a top portion and/or a sidewall portion of the stomach wall to prevent distal migration of the deployed balloon in a direction toward a pyloric sphincter. The sidewall attachments may comprise about three or four anchors circumferentially spaced and separated toward the proximal or top of the stomach to further minimize risk of distal migration.

In an embodiment, the introducing step comprises fluidically connecting a fluid cannula to the gastric balloon interior volume through a transabdominal wall of the patient such as via the cannula working channel, or through an endoscope, the fluid cannula having an outer end positioned outside the patient; and introducing a fluid to said gastric balloon internal volume via the fluid cannula outer end, thereby increasing the gastric balloon internal volume, including to the deployed state so as to occupy at least 75% of the stomach lumen.

The method may further comprise the step of introducing a malabsorption sleeve to at least a portion of a small intestine of the patient, wherein a malabsorption sleeve inlet is positioned at or adjacent to a pyloric sphincter and substantially aligned with an exit of the annulus.

In an aspect, the anchoring step further comprises placing one or more pre-sutures or preclosure sutures through an anchor mount opening, so that during tissue wall defect closure with the sutures during removal of the gastric cannula, the anchor is also secured to the gastric wall. Such a configuration allows fast and reliable wall closure and anchoring in a single step.

As part of the weight loss device to which the balloon is anchored, accessed, and/or inserted into the body, provided herein is a trans-abdominal gastric surgical system or, more generally, a transabdominal gastric cannula that provides access to a patient's abdominal cavity from outside the patient through the abdominal wall with a uniquely inserted, secured and removable cannula, including any of the systems described in U.S. Pat. App. No. 14/451,108 filed Aug. 4, 2014. The unique structure and implementation of the systems described herein provide a number of important functional benefits that increase the likelihood of a successful outcome and minimizes risk of an adverse event. First, the gastric cannula is readily and easily deployed, with a retrograde introduction from the oral-pharynx, esophagus, to the stomach lumen, and into the abdominal wall, with an internal anchor in contact with an inner-facing surface of the stomach wall by a simple pulling force on a guidewire connected to the system that passes through an incision in the abdominal wall. Once in place, the cannula portion is readily anchored through the use of opposed anchors, an internal anchor that is anchored to the gastric or peritoneal surface and an external anchor opposed to the internal anchor that is anchored to the skin surface. This results in an extremely reliable and robust positioning of the cannula, through which a surgeon can access internal regions of a patient, including a gastric balloon, anchors, and the lumen-facing portion of the stomach wall. Furthermore, a fluid conduit can be readily positioned through the lumen of the transabdominal gastric cannula to provide controlled inflation and/or deflation of the gastric balloon. Closure elements may be inserted at the start of the procedure, making system removal and incision closure simple, quick and reliable with an attendant decrease in scarring-related issues, tissue sensitivity, pain, infection and wound re-opening. In particular, the closure elements may be integrated with the anchor, so that upon tissue closure, balloon anchoring to the stomach wall is simultaneously achieved in a rapid, reliably and robust manner.

Optionally, the balloon is preconnected to the gastric cannula ahead of a retrograde introduction, such as to the internal anchor prior to gastric cannula introduction to the patient, so that after the gastric cannula is introduced the balloon is prepositioned in an undeployed state in the stomach lumen. Alternatively, the balloon is inserted into the stomach lumen after the gastric cannula is placed in the patient, such as via an endoscopic introduction through the esophagus or a laparoscopic introduction via the gastric cannula. The balloon may then be inflated from an undeployed to a deployed configuration by introduction of a fluid to the balloon lumen. The working channel(s) of one (or more than one) gastric cannula is an important aspect that facilitates precise and reliable balloon anchoring to the gastric wall, and attendant functional benefits.

The systems are compatible with a wide range of applications including related to the ability to manipulate tissues, manipulate and anchor implanted devices such as balloons and/or sleeves, provide internal sutures, and accommodate a variety of anastomotic devices. An application of interest herein, is weight loss via balloon inflation in the stomach lumen and the attendant medical instruments introduced through the gastric cannula, such as graspers and manipulators for balloon and/or sleeve positioning and anchoring. Further advantages include the ability to maintain a desired spatial orientation during a surgical procedure as well as providing the ability to multitask via the introduction of a plurality of medical instruments that do not interfere with each other. If there is an adverse event during a surgical procedure, the systems herein allow for rapid management and mitigation, including any of a number of intraperitoneal complications such as a hemorrhage event, balloon failure, or the like. Other advantages include the ability to triangulate on a specific region of interest in either a one-cannula or two-cannula configuration. This is particularly useful for the balloon anchoring aspect, where it is otherwise impractical to manipulate the balloon, anchor and pre-sutures. Furthermore the systems provided herein are compatible with any generic endoscopic or laparoscopic instrument to provide a degree of control not previously possible. Such control is an important part of ensuring appropriate balloon positioning.

The minimally invasive and low complication incidence provided by the instant systems means unwanted physiological events are avoided along with attendant decrease in training efforts and costs, with both improved patient outcomes and decreased healthcare costs that are otherwise associated with failure to address obesity adequately. The simplicity and elegance of the systems ensure a rapid learning curve is achieved by a spectrum of caregivers.

In an embodiment, provided herein is a trans-abdominal gastric surgical system that can be used with any of the balloons provided herein.

In use, the transabdominal gastric system provides two opposed surfaces connected to and securing the cannula to the abdominal wall, with the internal anchor securing the system in a direction from the gastric environment to the abdominal wall, and a counter-directed force from the external anchor toward the abdominal wall.

The systems and related methods provided herein are a useful platform for a number of applications. Depending on the application of interest, the system accommodates a medical instrument that extends from outside the body to inside the body, via the working channel or cannula passage. Examples of medical instruments include, but are not limited to, laparoscopic and endoscopic instruments for minimally invasive surgery such as for tissue incision, removal, handling, illumination, surgery, suturing, stapling and the like.

In an embodiment, the internal anchor surface shape is adjustable, deployable, or both. Examples include an internal anchor selected from the group consisting of: a balloon; a hinged umbrella; and a flexible bumper. In a more basic implementation, the internal anchor surface does not substantially change shape, but instead is shaped and sized to permit endoscopic introduction from the mouth to the stomach while maintaining the ability to reliably secure the device against an internal-facing surface, such as the gastric wall or a peritoneal surface.

In an aspect, the internal anchor encircles the cannula inner end and is configured to secure the system to an interior surface of a gastric wall or an interior surface of a peritoneal cavity. In an embodiment, the internal anchor comprises a bumper and the bumper and the cannula are formed from a unitary material.

The bumper has a shape to provide reliable contact with a patient's inner-facing surface, including the stomach wall or a peritoneal cavity surface. For example, the curved outer surface may have a maximum diameter that is greater than or equal to 2 cm and less than or equal to 4 cm, a height that is greater than or equal to 0.5 cm and less than or equal to 2.5 cm, an open exit having a diameter that is less than or equal to 3.5 cm; and a hollow interior volume defined by said curved outer surface and through which a medical device can traverse. The hollow interior volume advantageously provides a well-defined space through which a medical device extends, thereby assisting with medical device control and positioning to enhance stability, while minimizing adverse effects on surrounding tissue during medical device insertion and removal and during an extended medical procedure.

Any of the systems provided herein may have an external anchor that comprises a disc having an inner-facing surface that defines a passage for receiving the cannula. In this aspect, the cannula and inner-facing surface may have a circular shape to provide a translational connection by a matched internal thread and external thread pair on facing surfaces of the disc inner-facing surface and the cannula outer-facing surface, wherein rotation of the disc relative to the cannula outer-facing surface translates the disc along at least a portion of the cannula outer-facing surface in a longitudinal direction along the cannula axis. Alternatively, the translational connection may comprise other connections, such as a friction-fit, clamp fit, or set screw fit.

In an aspect, the disc comprises a central body that defines the passage and a flange connected to the central body, the flange comprising a plurality of passages extending there through. The plurality of passages may be arranged in a circumferential offset pattern relative to a central body having a substantially circular shape. Adjacent passages may be separated by a separation distance that is greater than 1 mm and less than 4 mm and have alternating separation distances from the central body corresponding to a minimum separation distance and a maximum separation distance. For example, the minimum separation distance may be less than about 7 mm and the maximum separation distance greater than about 7 mm.

The flange may have an outer edge that comprises a plurality of straight edges, such as an octagon shape and corresponding number of passages. For example, the plurality of passages may number eight, with four corner-positioned passages and four side-positioned passages. Adjacent corners may be separated by an individual side-positioned passage, with the corner-positioned passages separated from the central body by the maximum separation distance and the side-positioned passage separated from the central body by the minimum separation distance. In an aspect, each of the plurality of passages is positioned adjacent to a corner region of the octagon shape flange outer edge. Each of the passages may be positioned within about 1 cm from the edge of the flange. In an aspect, each of the edges that define the flange outer edge has equal lengths. For an octagon embodiment, the edges may have a length that is between about 1 cm and 2 cm and accordingly spaced from an outermost edge of the central body of between about 0.5 cm and 1.5 cm. The maximum length of the disk may be between about 2 cm and 5 cm.

Any of the systems provided herein may further comprise a plurality of suture threads, wherein each individual suture thread traverses a pair of opposed passages, and may loop around an outermost portion of the internal anchor, without adversely impacting any of the one or more medical devices extending there through. For example, the suture threads may be positioned at the start of a procedure, the procedure performed, medical devices removed, and the suture threads pulled to remove the system from the patient and to reliably close an incision, as explained hereinbelow. Preferably, the presutures are operably connected to the gastric balloon anchor, as described herein, so that defect closure also results in balloon anchoring to the gastric surface.

Any of the systems provided herein may further comprise a cap removably connected to the cannula outer end. Such a cap is useful to providing an air-tight passage for introduction of medical instruments through the cannula from outside a patient so that a distal end of the medical instrument is provided inside the patient and extending past the internal anchor. For balloon volume control via inflation/deflation, a useful type of medical instrument is a fluid conduit fluidically connected to the balloon that introduces and/or removes a material from the balloon lumen, thereby controlling balloon volume

The cap may comprise one or more instrument ports configured for introducing one or more medical instruments to the cannula lumen and out of the inner end and into a patient when the system is anchored to a gastric wall or a peritoneal surface by the internal anchor and a skin surface by the external anchor.

The cap may comprise a plurality of instrument ports formed from a memory sealant, each instrument port having an independently selected size and introduction angle. The memory sealant may be a shape memory polymer. The memory sealant may be formed from a single layer or a multilayer. In such a manner, upon removal of the medical instrument, the memory sealant may form an airtight seal between the external and internal side of the sealant.

In an aspect, any of the systems may further comprise a pressure port operably connected to the cap for measuring or controlling pressure at the cannula inner end or in a balloon that is fluidically connected to the pressure port. In this manner, the balloon lumen may be pressurized to a desired pressure, such as to achieve a desired shape and volume, including to occupy a substantial fraction of the stomach lumen, such as greater than 75% in a desired position..

In an aspect, any of the systems may further comprise a stopcock connected to the cap for providing controlled access to the cannula lumen or a fluid conduit connected thereto.

Any of the systems provided herein may also be configured for introduction or insertion into the gastric lumen, such as by a retrograde introduction to the stomach from the esophagus. Accordingly, the system may have an external anchor removed configuration for the external anchor removed from the cannula. In this aspect, the system may further comprise an introducer removably connected to the cannula outer end in the external anchor removed configuration. Such external anchor removal may ready the system for insertion into a patient.

In this aspect, the introducer may comprise a receiving opening that removably receives the cannula outer end and at least a portion of said cannula central portion. The connection may be equivalent to the connection employed with the external anchor-cannula connection.

In an embodiment, the introducer comprises a distal end; a proximal end through which the receiving opening traverses; a tapered central portion extending between the distal end and the proximal end; a capture element connected to the distal end; and wherein the tapered central portion is configured for introducing the system to a patient by retrograde introduction past a patient's oropharynx by pulling a guidewire connected to the capture element in a direction away from the introducer connected to the system.

In an embodiment, the introducer and cannula have a flexibility or bending moment selected so that the introducer is capable of deforming to follow contours of a patient oral-pharynx and esophagus during insertion in a patient.

Once the introducer is pulled through the guidewire insertion region, the introducer is removed to provide a system having an introducer removed configuration. The system in an introducer removed configuration is then ready to receive an external anchor that is connected to the cannula outer surface in an external anchor deployed configuration.

In another embodiment, the invention is an insertable trans-abdominal gastric surgical system comprising an introducer having a receiving passage and an outer tapered surface; a cannula having an outer end removably connected to the introducer receiving passage; an inner end; and a central portion having an outer-facing surface that extends between the outer end and the inner end and an inner-facing surface that defines a lumen configured to receive a portion of a medical instrument that traverses between the outer end and the inner end. An internal anchor is connected to the inner end and has a surface shape configured to secure the system against an interior surface of a gastric wall or peritoneal surface and to optionally provide a tether point for any of the gastric balloons described herein to facilitate balloon positioning and placement. The introducer, cannula and internal anchor are configured for insertion to a patient's gastric lumen by retrograde introduction past a patient's oropharynx, along an esophagus, and into the stomach lumen.

Any of the systems provided herein may have an introducer that comprises a distal end; a proximal end through which the receiving passage traverses, wherein the receiving passage has an at least partially threaded inner-facing surface to rotationally and removably engage an at least partially threaded cannula outer facing surface.

Any of the systems provided herein may have an introducer further comprising a capture element connected to the distal end configured to connect to a guidewire to facilitate guided insertion in a direction through a patient's oropharynx, esophagus, stomach and abdominal wall, such as by a pulling action of the guidewire that is connected to the capture element away from the system. In this manner, the capture element then transmits the pulling action to the rest of the system, thereby moving the entire system.

In an aspect, the invention may be further described in terms of the outer tapered surface having: an angle of incidence at the distal end that is greater than or equal to 5° and less than or equal to 20°; a total length that is greater than or equal to 2 cm and less than or equal to 15 cm; a tapered portion extending from the distal end and a substantially untapered portion extending between the proximal end and the tapered portion, having a tapered portion longitudinal length to untapered portion longitudinal length ratio (L_{T}/L_{U}) that is greater than or equal to 1 and less than or equal to 5; and wherein the introducer has a flexibility selected so that said introducer is capable of deforming to follow contours of a patient's oral-pharynx and esophagus during insertion into a patient.

In an aspect, the cannula portion of the system may be further described in terms of certain dimensions, such as length and diameter. For example, the cannula length may be between 4 cm and 30 cm, and any sub-ranges thereof, such as between 6 cm and 10 cm. The cannula diameter may be selected from a range of between 5 mm and 70 mm, including a diameter that is greater than or equal to 5 mm and less than or equal to 20 mm, and any sub-ranges thereof. The introducer diameter may be accordingly sized to match the cannula diameter, such as a diameter of between 5 mm and 70 mm, or between 5 mm and 20 mm, and any sub-ranges thereof.

The introducer and/or cannula may be made from a material having a desired durometer, rigidity and flexibility, such as medical grade silicone, polyvinyl chloride (PVC), plastic, rubber, or other material known in the art of medical devices and implants.

Also provided are various methods related to any of the systems described herein. In an embodiment, provided is a method of inserting a trans-abdominal gastric surgical system in a patient by inserting a guidewire through an abdominal wall insertion and into a stomach lumen; guiding a portion of the inserted guidewire out of the stomach lumen, through an esophagus and mouth to provide an accessible portion of the guidewire; connecting a capture element of an introducer trans-abdominal gastric surgical system assembly to the accessible portion of the guidewire; pulling the guidewire connected to the capture element of the introducer trans-abdominal gastric surgical assembly in a direction away from the patient so the assembly is introduced into the stomach lumen; advancing the introducer portion of the assembly out of the stomach through the abdominal wall incision so that an internal anchor of the trans-abdominal gastric surgical system contacts an inner-facing surface of the stomach; removing the introducer from the assembly to reveal an exposed end of trans-abdominal gastric surgical system; removing the guidewire; connecting an external anchor to the exposed end of the trans-abdominal gastric surgical system; and moving the external anchor in a direction toward a skin surface of the patient to reliably secure the trans-abdominal gastric surgical system to the patient. In this manner, the trans-abdominal gastric surgical system is inserted and reliably secured to the abdominal wall of the patient.

In an aspect, the method further comprises the step of attaching a cap to the exposed end of the inserted trans-abdominal gastric surgical system. Alternatively, the system may be configured to have a self-contained cap over which the introducer extends, so that upon removal of the introducer, the cap is revealed.

The method is further useful for a variety of surgical procedures, including a procedure on a human or a non-human animal. For example, the method may further comprise the step of introducing one or more than one surgical instruments through the trans-abdominal gastric surgical system for use in a procedure selected from the group consisting of: weight loss via gastric balloon insertion in the gastric lumen; malabsorption sleeve in the upper GI tract or small intestine region; instrument triangulation; accessing a stomach lumen; accessing a retroperitoneal space; manipulating tissue; closing an incision; a gastric surgery; a gall bladder surgery; single or simultaneous access to an upper GI tract and small intestinal lumen; access of an intra-peritoneal space; and access of an extra-peritoneal space and associated organs, or any combination thereof.

In an aspect, any of the methods further comprise the step of removing the trans-abdominal gastric surgical system after procedure completion in a reliable, simple and robust manner that minimizes post-operative discomfort or complications, and that simultaneously anchors the balloon to the stomach wall. The removal may comprise inserting at a start of or before the procedure a plurality of sutures by: introducing a cannulated-introducer needle through a first passage in the external anchor and through a first underlying tissue region comprising an abdominal and gastric wall and into a gastric environment, either for a single-layer closure or in a repeated manner for multiple-layer closure; introducing a suture grasper through a second passage in the external anchor and through a second underlying tissue region comprising the abdominal and gastric wall and into the gastric environment, wherein the second passage is opposibly positioned relative to the first passage; placing a suture thread proximal portion through the cannulated-introducer needle; guiding a suture thread distal portion around and away from the outer-facing surface of the internal anchor to ensure there is no interference by the thread on movement and use of a medical instrument passing therethrough and guiding the suture thread through the anchor mount opening, wherein the suture thread distal portion longitudinally extends from the suture thread proximal portion; grasping at least a portion of the suture thread distal portion with the suture grasper; pulling the suture grasper and suture thread distal portion out of the gastric environment. through the anchor mount opening, and through the underlying abdominal and gastric wall and the external anchor second passage wherein the suture thread portion in the body is positioned around or beyond said internal anchor to avoid interference with an instrument introduced through a working channel formed by the trans-abdominal gastric surgical system, and securing the suture ends outside the body to ensure the suture thread ends are not pulled back into the patient, such as by clamps that lay externally to the patient, thereby providing a reliable pre-closure suture; repeating the above steps with a second suture thread positioned through a third external anchor passage, fourth external anchor passage, and corresponding third and fourth underlying tissue regions comprising the abdominal and gastric wall; optionally, the steps are repeated with a third suture thread and fifth and sixth external anchor passages, as desired and depending on the number of available external anchor passages. The cannulated-introducer needle and the suture grasper are removed to reveal matched pairs of suture thread proximal and distal portions that extend out past the external anchor outside the patient skin surface and that each traverse through the anchor mount opening; the external anchor is loosened or optionally removed from the exposed end of the trans-abdominal gastric surgical system; the revealed matched pairs of suture thread proximal and distal portions are pulled in a direction away from the patient, thereby removing the trans-abdominal gastric surgical system from the patient; and closing the incision by suturing the suture threads in a position that is outside the abdominal wall thereby closing abdominal wall incision and securing the anchor, and thereby the gastric balloon, to the gastric surface.

Without wishing to be bound by any particular theory, there may be discussion herein of beliefs or understandings of underlying principles relating to the devices and methods disclosed herein. It is recognized that regardless of the ultimate correctness of any mechanistic explanation or hypothesis, an embodiment of the invention can nonetheless be operative and useful.

### BRIEF DESCRIPTION OF THE DRAWINGS

**FIG. 1****.** Perspective view of a trans-abdominal gastric surgical system without an external anchor.
**FIG. 2****.** Views of a trans-abdominal gastric surgical system with external anchor and cap showing the interior volume of the internal anchor (top panel), the instrument port (middle panel) and a side view (bottom panel).
**FIG. 3****.** A trans-abdominal gastric surgical system and connector element ready to receive a guidewire for insertion into a patient (left panel). The right panel is a close-up view of the capture element connected to the introducer distal end.
**FIG. 4****.** Deployed annular balloon connected to a single fluid conduit and tethered to a subcutaneously placed port for inflation or deflation of the balloon.
**FIG. 5****.** Deployed annular balloon connected to one fluid conduit and one transabdominal gastric cannula.
**FIG. 6****.** Deployed annular balloon anchored to the stomach wall, with malabsorption sleeve, and the transabdominal gastric cannula removed from the patient.
**FIG. 7A-7C****.** Schematic illustration of a gastric balloon anchor and presutures for reliably anchoring a gastric balloon to a gastric wall. **FIG. 7A** illustrates use of the transabdominal gastric cannula that can provide pre-sutures through an anchor mount. **FIG. 7B** illustrates how the presutures may be used to pull the gastric anchor toward the gastric wall to secure the balloon to the gastric wall via the anchor. **FIG. 7C** shows a three-ring anchor embodiment, and the anchor that is secured within the gastric wall by the sutures.
**FIG. 8****.** Two transabdominal gastric cannula with medical instruments disposed therethrough, and undeployed gastric balloon in the gastric lumen and unextended elongated sleeve in the lumen of the upper GI tract. Also shown is an endoscopic instrument, such as a fiber optic light source.
**FIG. 9** illustrates use of medical instruments for connecting anchors to the gastric wall. Inset is a view down the lumen of the pyloric sphincter illustrating four anchors.
**FIG. 10****.** The system of **FIG. 9****,** illustrating instrument triangulation on an anchor to anchor the balloon to the gastric wall.
**FIG. 11****.** Partial balloon deployment in the stomach and elongated malabsorption sleeve deployed in the duodenum connected via a tether to the transabdominal gastric cannula.
**FIG. 12****.** Deployed non-annular gastric balloon with an elongated gastric sleeve connected thereto.
**FIG. 13****.** Flow chart summary of a method of removing the system and securing the gastric balloon anchor to the gastric wall; and as schematically illustrated in **FIGs 7A-7C** and **14-17.**
**FIG. 14****.** Schematic illustration showing suture thread introduction through a first external anchor flange passage.
**FIG. 15****.** The suture thread of **FIG. 14** is grasped and pulled out of the patient with a grasper that traverses a second external anchor passage that is opposibly positioned relative to the first external anchor passage. Optionally, suture guide elements assist with positioning of suture threads in a desired linear position over the internal anchor outer-facing surface.
**FIG. 16****.** The steps outlined in **FIGs 14-15** are repeated for a second suture thread.
**FIG. 17****.** The accessible suture threads are pulled so that all suture threads and the internal anchor are pulled outside the abdominal wall, thereby suturing the incision closed. The inset illustrates the opposing passages configuration through which suture threads pass.
**FIG. 18****.** Flow chart summary of system introduction to a patient and corresponding to illustrations in **FIGs 19-22****.**
**FIG. 19****.** Guidewire insertion through the abdominal and gastric wall, up the esophagus and out of the patient mouth.
**FIG. 20****.** Guidewire connection to an introducer-system and start of retrograde introduction at the patient's mouth
**FIG. 21****.** Continued retrograde introduction of the introducer-system to the gastric environment and positioning the system by pulling the guidewire out of the abdominal incision and forcing the introducer through the abdominal wall incision
**FIG. 22****.** External anchor and cap connection and positioning of the external anchor against an outer surface of the patient to provide reliable positioning of the system and a reliable working channel through the abdominal wall. Also illustrated is an endoscope having a fiber optic light source.

### DETAILED DESCRIPTION OF THE INVENTION

In general, the terms and phrases used herein have their art-recognized meaning, which can be found by reference to standard texts, journal references and contexts known to those skilled in the art. The following definitions are provided to clarify their specific use in the context of the invention.

"Obesity" refers to a patient that is diagnosed as overweight, including morbidly obese or at least non-morbidly obese, including as being overweight with a body mass index in a range of about 30-35, and is desirous of losing weight.

"Treating" refers to the use of the systems described herein that are functionally equivalent to gastric restriction via the use of a deployable gastric space occupying device described herein as a size-varying gastric balloon. The balloon is inserted into the patient in an uninflated state, and subsequently inflated to occupy a substantial fraction of the stomach lumen.

"Low profile" refers to transabdominal gastric cannula having minimal protrusion out of the patient and/or into the stomach lumen. In an embodiment, the protrusion is less than 1 cm, or less than 5 mm from a corresponding surface.

"Proximal stomach portion" refers to that portion of the stomach which is between the esophagus sphincter and the closest point of the gastric balloon, or to the annulus entry.

"Distal stomach portion" refers to that portion of the stomach which is between the pyloric sphincter and the closest point of the gastric balloon, or to the annulus exit.

"Constrained movement" refers to the balloon, and portions thereof, having a degree of movement to accommodate the ebb and flow of peristalsis, but that ensures the prevention of balloon distal migration and attendant side-effects related thereto. In contrast, balloons that are substantially constrained with a rigid and stationary anchor tend to suffer gastric wall irritation and other side effects, thereby decreasing long-term efficacy.

"Long term use" refers to a time period sufficient to achieve a measureable decrease in patient weight, such as for a time period on the order of weeks or months. An important aspect of the balloon systems provided herein is the ability to reliably position the balloon in place to avoid the drawbacks of conventional balloon systems that tend to migrate toward the pyloric sphincter, such as during peristaltic movement of food through the GI-tract. The systems provided herein, including specially constructed gastric balloon anchors in combination with the transabdominal gastric cannula, facilitate reliable balloon anchoring to the gastric wall to avoid such unwanted migration.

"Bumper" refers to a shape of the internal anchor outer surface that is substantially curved and configured to provide reliable contact with an inner surface of a biological tissue, such as the stomach wall or peritoneal surface.

An "internal anchor" of the present invention is the element that is positioned in the body and that anchors the device to an inner surface of the body, in combination with the external anchor. "Adjustable" or "deployable" internal anchor surface shape refers to an internal anchor that can be adjusted or actuated from a first state or shape to a second state or shape. For example, a balloon-type internal anchor can have a surface shape that is adjustable by varying the pressure in the internal volume encompassed by the balloon surface or deployable by inflating from an uninflated state. An umbrella-type mechanism may be adjusted to provide surface shape adjustability in curvature. This aspect also facilitates control of the total surface contact area between the internal anchor surface and the corresponding biological surface, including the stomach or peritoneal wall.

A gastric balloon "anchor" refers to the element that reliably and controllably connects the gastric balloon, specifically a deployed gastric balloon, to the gastric wall. As desired, multiple anchors can be used, with the anchors designed to provide constrained motion of the balloon while avoiding gastric irritation and unwanted balloon motion.

"Flexible" refers to shape deformation under an applied force. Accordingly, a flexible anchor or flexible bumper refers to an anchor or bumper whose shape can at least partially conform to increase the magnitude and reliability of the contact area between the anchor and the corresponding biological surface. A flexible introducer, refers to the ability to deform in order to navigate the contour of a patient's anatomy for system introduction. One quantitative indication of flexibility is Young's modulus (defined as stress/strain). In an aspect, the introducer is formed of a polymer material having a Young's modulus that is less than or equal to 10 GPa, less than or equal to 10 MPa, or less than about 1 MPa, or any other range that provides the desired functional outcome of system flexibility during introduction along the esophagus.

Similarly, the dimensions and geometry of the introducer in combination with the material properties may provide a bending moment useful for system introduction to the gastric environment via the esophagus. "Bending moment" refers to the force on a portion of the introducer that generates a corresponding deflection of the introducer. The bending moment may be quantified based on a cantilever approximation, with an introducer having one end fixed, such as the proximal end that is connected to the cannula, and the other end such as the distal end that is free to move. The bending moment is selected so that a typical force experienced when introducing the system into a patient to follow contours of the esophagus, the system correspondingly bends or deflects to follow the contours and facilitate introduction and minimize unwanted forces on the esophageal wall.

"Removably connected" refers to a configuration of elements, wherein the elements can be temporarily connected to each other and, as desired, removed from each other without adversely impacting the functionality of other elements of the device. "Translationally connected" refers to a configuration of elements, wherein motion of one element is substantially unidirectional and parallel with respect to another element, wherein movement of one element does not affect each element's functionality. "Operably connected" refers to a configuration of elements, wherein an action or reaction of one element affects another element, but in a manner that preserves each element's functionality. For example, a pressure port operably connected to a cap refers to the ability to monitor or effect pressure change without impacting the functionality of the cap, including having other ports for introduction of medical instruments.

"Fluidically connected" refers to a configuration of elements, wherein a fluid (e.g., liquid, gas or viscoelastic material) in one element is able to enter another element in a manner that does not affect each element's functionality. For example, a fluid conduit that is fluidically connected to a balloon does not affect the balloon's ability to be positioned and inflated/deflated.

"Unitary material" refers to two elements that are integrally connected, such as an internal anchor and cannula that are formed from one piece. This is in contrast to identical material that may be more permanently connected, such as by an adhesive or bond, or removably connected such as by a threaded connection.

"Capture element" refers to the portion of the introducer on which a force is exerted so as to advance the system into the patient, such as down the esophagus, into the gastric environment and through the abdominal wall so that one end of the system remains in the patient and the opposite end is accessible from the environment that is outside a patient's body. The invention is compatible with a range of capture elements, so long as the ability to reliably introduce the system by retrograde introduction via the esophagus is not impacted. Specific examples include, but are not limited to, a wire loop, a suture, a connection mechanism such as snap-fit, magnets, threaded attachment, clamp or fasteners.

"Fluid" refers to a material that may be removed or introduced from a volume to effect a change in pressure, including a material that flows under an applied force. Depending on the application of interest, the fluid may be a gas, a liquid, a gel, or a combination thereof.

Unless defined otherwise, "substantially" refers to a value that is within at least 20%, within at least 10%, or within at least 5% of a desired or true value. Substantially, accordingly, includes a value that matches a desired value.

### Example 1: Trans-abdominal gastric surgical system.

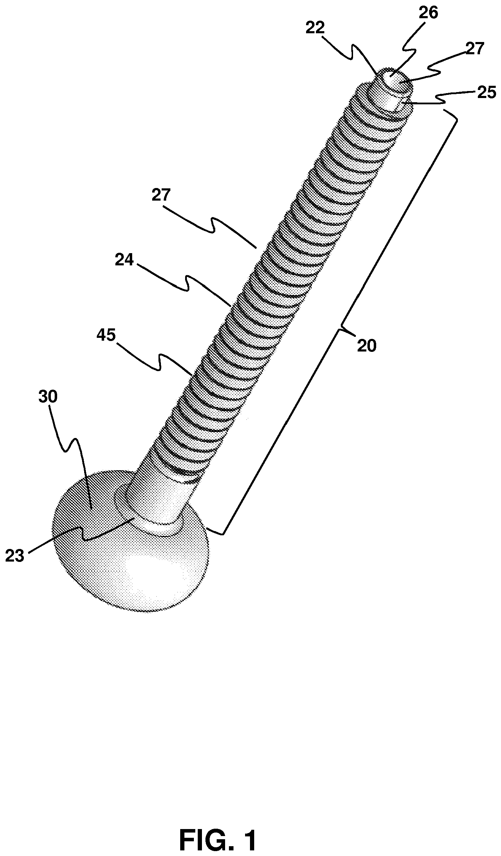

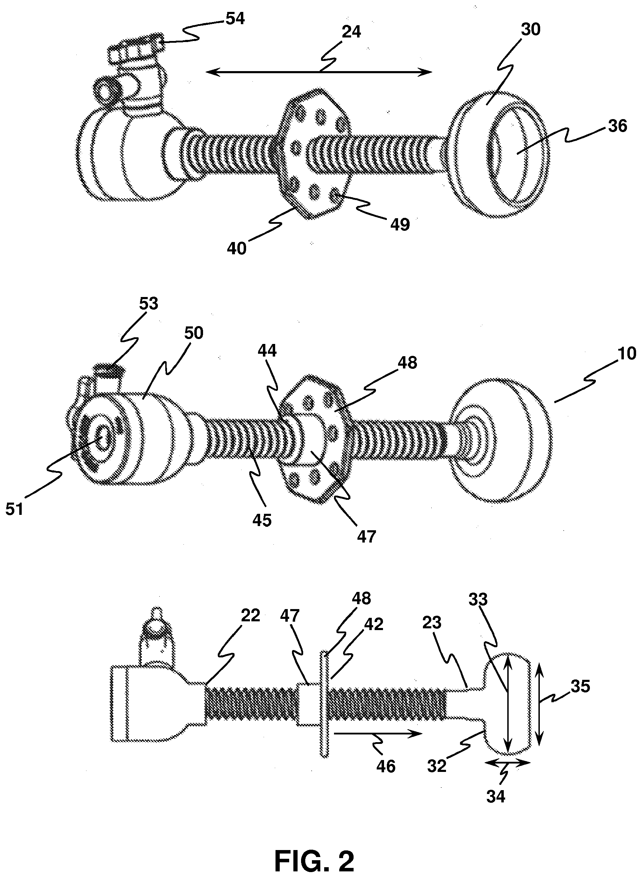

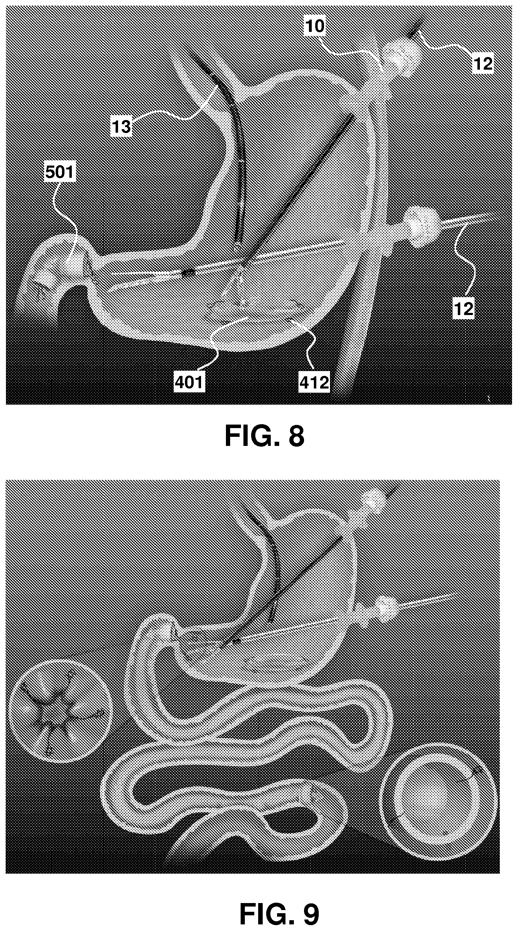

Referring to **FIGs. 1-3****,** a trans-abdominal gastric surgical system **10** or generally, system, has a cannula **20** with an outer end **22,** an inner end **23** and a central portion **24.** Central portion has an outer-facing surface **25** that extends between the inner end and the outer end and an inner-facing surface **26** that defines a lumen **27** configured to receive a portion of a medical instrument **12** (see, e.g., **FIG. 8**) that traverses between the cannula outer and inner ends, such as from outside the patient to inside the patient. Lumen **27** is also referred herein as a working channel during use, such as a working channel to the stomach lumen. An internal anchor **30** is connected to the inner end **23** and has a surface shape **32** configured to secure the system against an interior surface of a gastric wall or peritoneal surface. An external anchor **40** is removably and translationally connected to the cannula outer-facing surface and has a surface shape **42** configured to secure the system against a skin surface **(****FIG. 2****).** Accordingly, **FIG. 1** illustrates an external anchor removed configuration, because external anchor **40** is not connected. This is in contrast to **FIG. 2****,** where external anchor **40** is connected and is capable of being positioned at various longitudinal distances along cannula axis, as indicated by arrow **46** directed along the system longitudinal axis.

As illustrated in **FIG. 2****,** the internal anchor may correspond to a bumper having a curved outer surface **32** with a maximum diameter **33,** a height **34,** an open exit diameter **35,** and a hollow interior volume **36.**

Referring to **FIG. 2** (external anchor connected configuration), external anchor **40** may be a disc having an inner-facing surface **43** that defines a passage **44** for receiving a cannula, more specifically cannula central portion **24.** The translational connection between external anchor **40** and cannula **20** may be a matched internal thread **28** and external thread **45,** illustrated as being on the inner-facing surface **43** of external anchor **40** and outer-facing cannula surface **25** of cannula **20,** respectively. In this manner, the external anchor may be positioned to have any desired separation distance from the internal anchor by moving along the cannula in a longitudinal direction, as indicated by arrow **46.** Other translational connections may be employed, including a tight friction fit, clamp, snap-fit, fasteners, set screws, or the like. The external anchor **40** may have a central body **47** in which the passage **44** is disposed and a flange **48** connected thereto. A plurality of second passages **49** may extend through the flange, for facilitating suture placement and system removal.

To provide controlled access to the cannula from the outer end **23** a cap **50** may be removably connected to the cannula outer end. The cap may have one or more instrument ports **51** through which one or more medical instruments **12** may be inserted. The instrument port **51** may be formed from a memory sealant material. A pressure port **53** may be connected to the cap **50** to control pressure, such as by removal or introduction of a fluid to cannula inner end **22,** and thereby to the gastric environment or a fluid conduit connected thereto which, in turn, is fluidically connected to a balloon. Controlled access to the cannula may also be provided by a stop-cock **54** type mechanism connected to the cap.

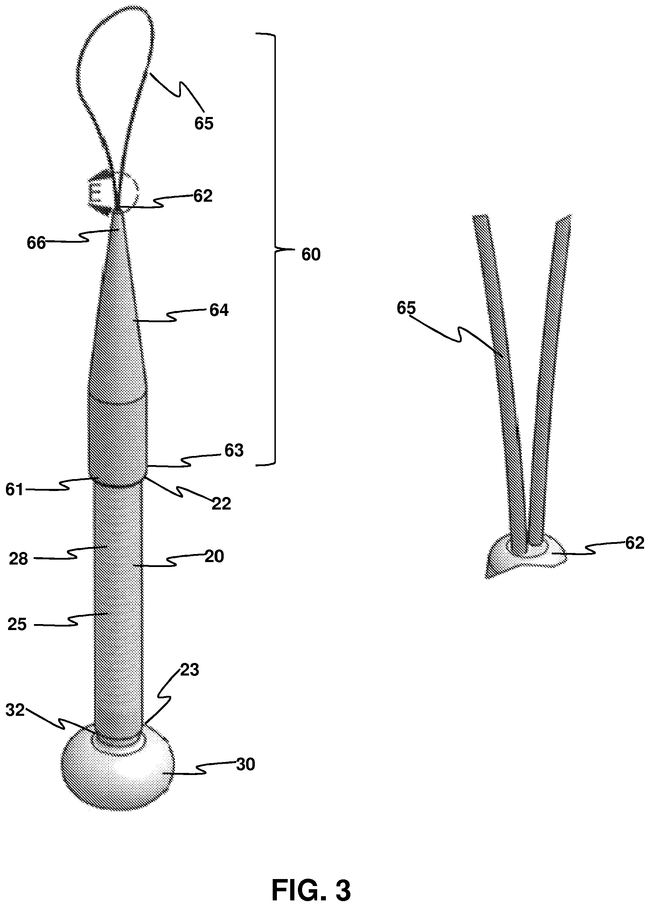

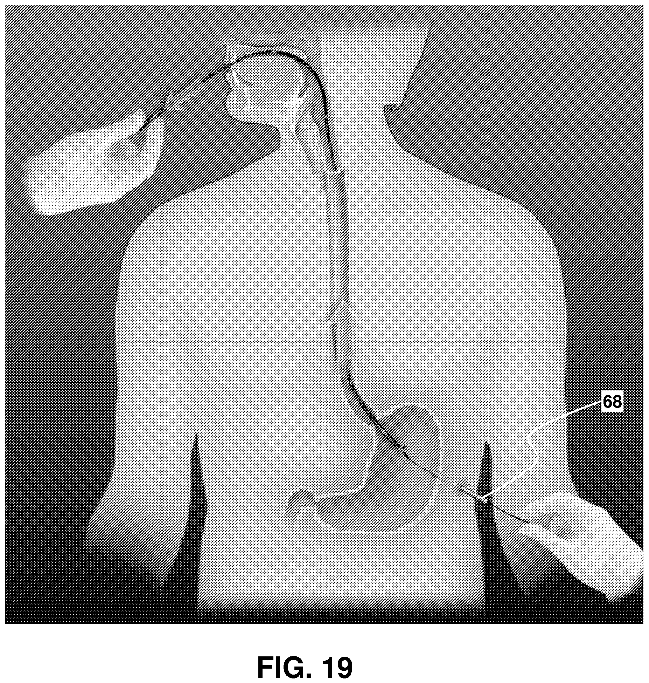

To facilitate system introduction to a patient, an introducer **60** may be used. Referring to **FIG. 3****,** introducer **60** is removably connected to the system, such as when the external anchor **40** is removed as shown in **FIG. 1****,** so as to provide a system that may be introduced to the gastric environment by retrograde introduction past a patient's oropharynx and into the gastric environment. Accordingly, **FIG. 1** may be further described as in an introducer-removed configuration and **FIG. 3** in an introduction ready configuration that is ready to be inserted into the patient by retrograde introduction. The introducer may have a receiving opening **61** that removably receives the cannula outer end **22** and a portion of the cannula central portion **24.** The receiving passage may have a threaded portion, such as to removably engage with the cannula central portion, in a manner similar to that of the external anchor and cannula central portion. A distal end **62** may connect to a capture element **65,** illustrated as a wire loop. A proximal end **63** may contain the receiving opening **61,** and a tapered central portion **64** that extends between the distal **62** and proximal **63** ends. In this manner, a guidewire **68** (see, e.g., **FIG. 19**) may be used to pull the introducer and system combination into the gastric environment. The tapered end of the introducer may then be pulled through the incision in which the guidewire passes to provide a reliable contact area between the internal anchor and the gastric wall, as well as gently expanding the abdominal opening to ensure a good fit in the abdominal wall. Similarly, reliable contact area between the internal anchor and an inner facing surface of the peritoneal cavity may be established for an equivalent incision for a guidewire provided therein.

Once the system is positioned accordingly, the introducer may be removed to provide an introducer-removed configuration **67** that is ready to receive the external anchor **40** (see, e.g., **FIG. 23**)**.**

### Example 2: Size Varying Gastric Balloon

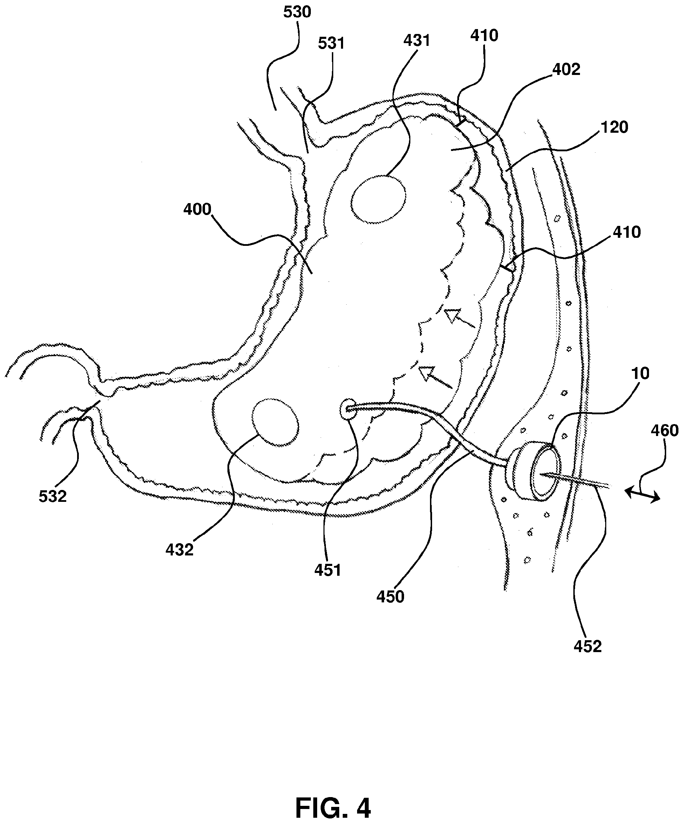

One example of a gastric balloon system for obesity treatment is illustrated in **FIGs 4-5****.** A size-varying gastric balloon **400** is provided with a trans-abdominal gastric cannula or subcutaneous port **10,** including the trans-abdominal gastric system discussed in Example 1 and **FIGs. 1-3****.** The balloon **400** has an internal volume **402** defined by balloon surface **410.** One or more gastric balloon anchors **412** connected to the balloon can connect to the gastric wall and/or the cannula **10** to reliably position and anchor the balloon in the gastric environment.

For balloon inflation/deflation, a fluid conduit **450** may traverse the gastric cannula **10** and fluidically connect to the balloon at a first fluid end **451** and have a second end **452** positioned externally to the patient. This facilitates introduction or removal of fluid, thereby controlling the shape of the balloon implanted in the stomach **120.** A fluid source for introducing and/or removing a fluid, such as a liquid or a gas, is indicated by arrow **460.**

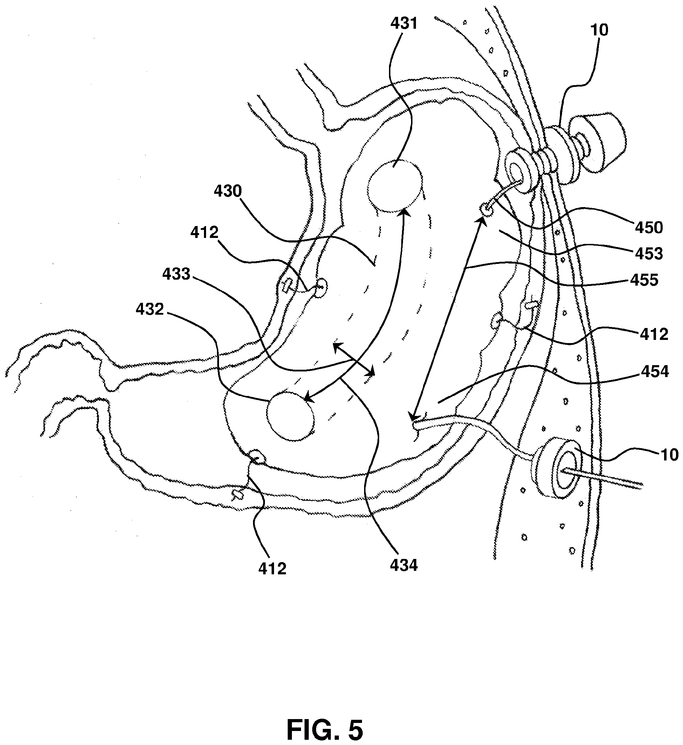

**FIG. 5** illustrates that the balloon anchor may correspond to a fluid conduit **450** connected to the transabdominal gastric cannula **10,** such as in an anterior portion **453** of the balloon and stomach wall. In this manner, the fluid conduit may functionally provide a tether type mechanism, thereby providing additional positional control of the balloon. In addition, a fluid conduit may connect to a cephalad portion **454** of the balloon. **FIG. 5** illustrates a two gastric cannula **10** and two fluid conduit **450** embodiment, with the first fluid ends separated by a fluid conduit separation distance **455.** In this manner, a plurality of inflation/deflation ports is provided. For example, during use one of the ports may be accessed endoscopically, as desired, particularly for embodiments where the gastric cannula and an associated percutaneous fluid cannula have been removed.

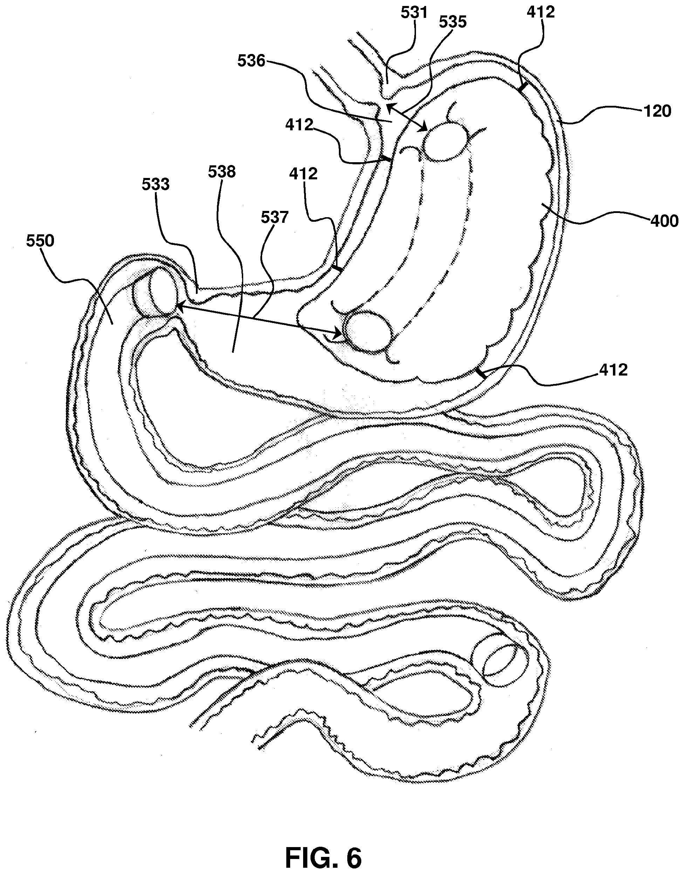

**FIG. 6** illustrates the embodiment where after balloon insertion and inflation, the transabdominal gastric cannula **10** (see, e.g., **FIGs 4-5**) may be removed, leaving the deployed and inflated gastric balloon **400** anchored to the stomach wall **120** by a plurality of anchors **412.** Alternatively, the gastric cannula **10** may remain in the patient. In those situations, the gastric cannula is provided in a low-profile to increase patient comfort.

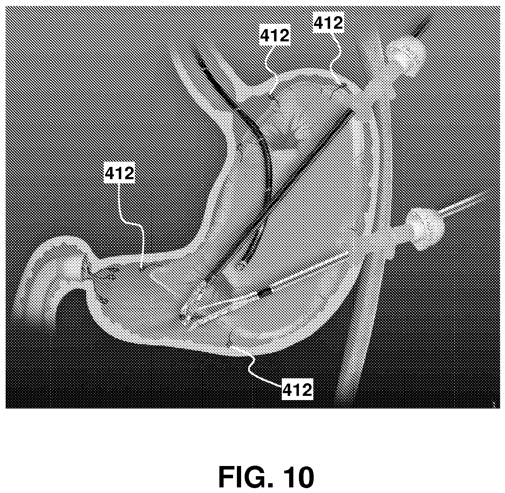

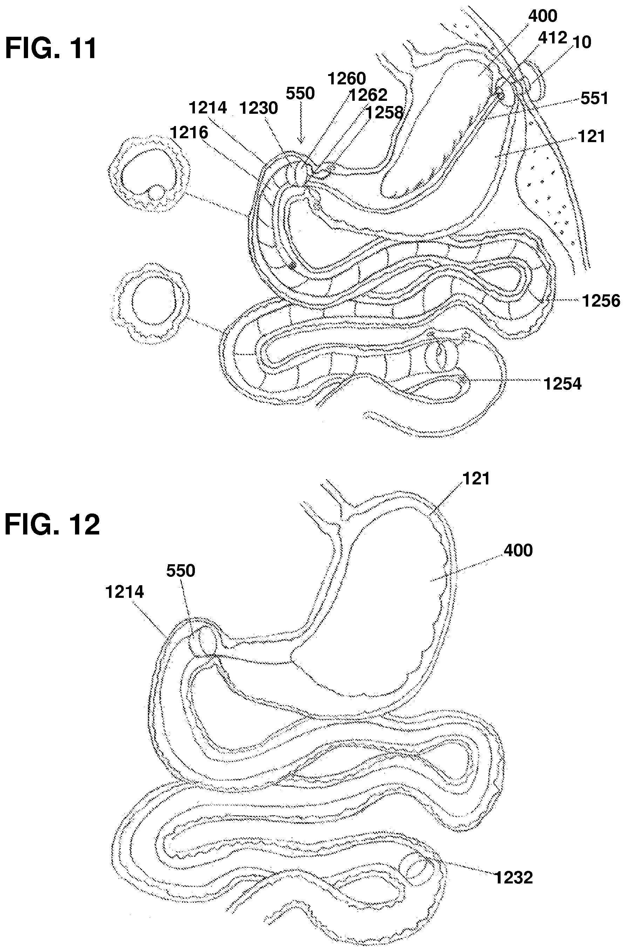

As illustrated in **FIGs. 11-12****,** the balloon **400** may have a non-annulus shape. Alternatively, the balloon may have an annulus **430,** as illustrated in **FIGs 4** and **10****.** Annulus has an entry **431** and exit **432** aligned with the esophageal **530** exit or sphincter **531** and the stomach exit or pyloric sphincter **533,** respectively. The annulus is configured to facilitate passage of fluids and food solids from the esophagus and out of the stomach and is optionally defined in terms of an average diameter and length, as indicated by arrows **433** and **434,** respectively **(****FIG. 5****).**

An important aspect of the systems and methods is the ability to position the balloon such that when it is deployed and during use, including long-term use on the order of weeks or months, including greater than 6 months to two years or more, the balloon's position is maintained within the stomach. Referring to **FIG. 6****,** a desired positon can be defined in terms of a separation distance from the esophageal sphincter **531** and from the pyloric sphincter **533,** as indicated by arrows **535** and **537,** respectively. An alternative description of the balloon position is provided in terms of volumes and fractional portions thereof. For example, when fully deployed, the balloon may occupy at least 75% of the stomach lumen. The unoccupied fraction of the stomach, however, is not uniform. The major unoccupied portions are in a proximal stomach portion **536** adjacent to the esophageal sphincter **531** and the distal stomach portion **538** adjacent to the pyloric sphincter **533.** Of those two portions, however, the distal stomach portion **538** has a substantially larger volume than the proximal stomach portion **536** . This is a reflection that the instant invention ensures there is sufficient separation distance from the pyloric sphincter to minimize risk of a gastric blockage.

Any of the systems provided herein may be used with an elongated sleeve , such as depicted by **550** in **FIG. 6****,** and as further explained in U.S. Pub. No. 2014/0276338.

Referring to **FIGs. 8-10****,** manipulation of the balloon and sleeve is illustrated via medical instruments **12** inserted through one or more transabdominal gastric cannulas. In **FIG. 8****,** illustrated is an undeployed balloon **401** and sleeve **501.** A medical instrument **12** through one of the transabdominal gastric cannula is used to secure sleeve anchors for sleeve deployment, as illustrated in **FIG. 9****.** As shown in **FIG. 10****,** the medical instruments **12** traversing the cannula of **10** are used to secure balloon anchors **412** to the gastric wall in strategic locations to ensure appropriate separation distances from the stomach entry and exit and corresponding appropriate volumes for proximal **536** and distal **538** stomach portions. This anchoring may occur prior to balloon inflation, after balloon inflation, during an intermediate semi-inflated balloon configuration where the balloon is only partially inflated, or any combination thereof.

**FIG. 11** illustrates a gastric balloon **400** in the stomach lumen **121** tethered to a transabdominal gastric cannula **10** via anchor or tether **412.** As explained further in U.S. Pub. No. 2014/0276338, tube or catheter **551** may be operably connected to elongated sleeve **550** positioned in the upper GI region of the small intestine having a proximal end adjacent to the pyloric sphincter secured to the gastric wall. Also illustrated, for clarity, is small intestine **1214,** malabsorption sleeve **1216,** collar member **1230,** central aperture **1260,** catheter passage **1262,** anchor mechanisms **1258,** distal anchor weight **1254,** and lumen support element **1256.** **FIG. 12** illustrates the sleeve **550** having a distal end **1232,** with the sleeve connected to a deployed balloon **400** and with the transabdominal gastric cannula **10** of **FIG. 11** removed. As discussed, the balloon **400** may be anchored to the gastric wall by one or more gastric balloon anchors.

The intra-gastric balloons (IGB) described herein are preferably smooth, seamless, and constructed of a material that is long-lasting, inert, non-toxic, having low ulcerogenic potential while being resistant to gastric acid. The balloon may have a radiopaque marker to assist with post-procedure imaging. The configuration of the systems provided herein ensures there is a low obstructive potential. The balloon is adjustable to a variety of sizes and can be filled with a fluid that is a liquid or air. A self-sealing valve may be incorporated to facilitate volume control and adjustment. The balloon is non-permanent and can be readily removed as desired, such as by endoscopic procedures with an endoscopic catheter.

### Example 3: Gastric Balloon Anchors

Also provided herein are gastric balloon anchors, including anchors that may be used with conventional balloons, or any of the balloons herein. For retrofitting, an anchor may be affixed to an outer surface of a balloon. Alternatively, balloons may be made with the anchor an integral part thereof, such as an anchor having one end embedded within or through the balloon wall.

The anchors may be manufactured such that they are an integral part of the balloon, including by having one end at least partially or fully embedded in the balloon wall. Alternatively, the anchors may be supplied as an after-market addition, thereby transforming conventional balloons into a balloon compatible with the systems and methods provided herein. The anchors are configured to have a certain amount of give at either end, to ensure there is not an over-tightness or rigidity at the site of attachment. For example, the anchor retains an ability to move in relatively small distances, including horizontally, vertically and/or rotationally. A small distance refers to the movement of the balloon that accommodates the ebb and flow of gastric motion, e.g., peristalsis, but not so much as to result in an unwanted distal migration of the balloon toward the pyloric sphincter. In this manner, the anchor may be incorporated in its attachment to the gastric wall by using the same closure technique as for wall defect closure during cannula removal. For example, the same sutures may serve two functions of: (1) defect closure; and (2) balloon anchoring to the gastric wall. The anchor, and attendant exposed sutures, are biocompatible and able to withstand the acid environment associated with the stomach lumen. There are many possible anchor designs that achieve this function.

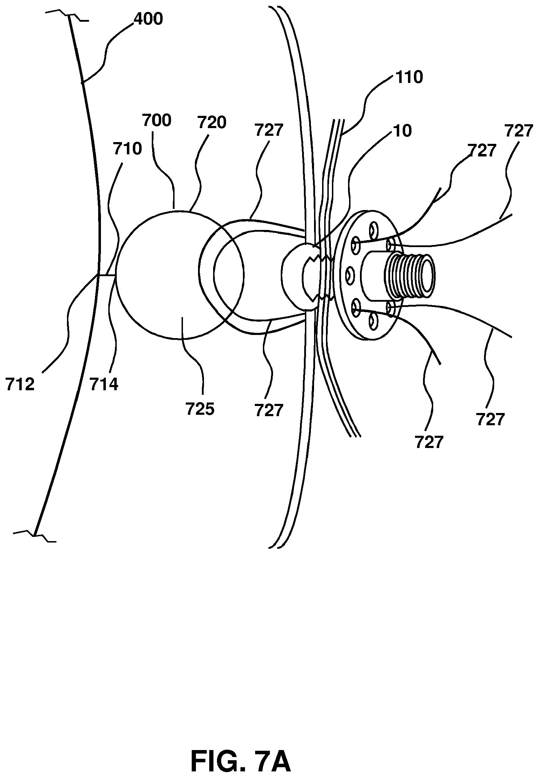

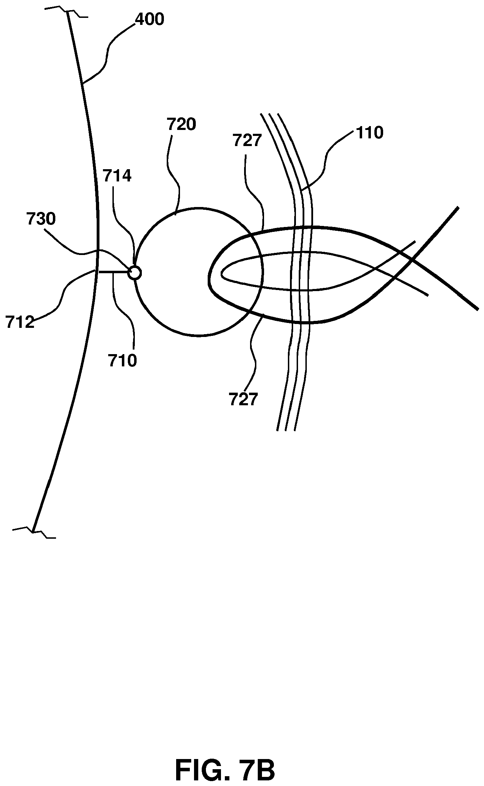

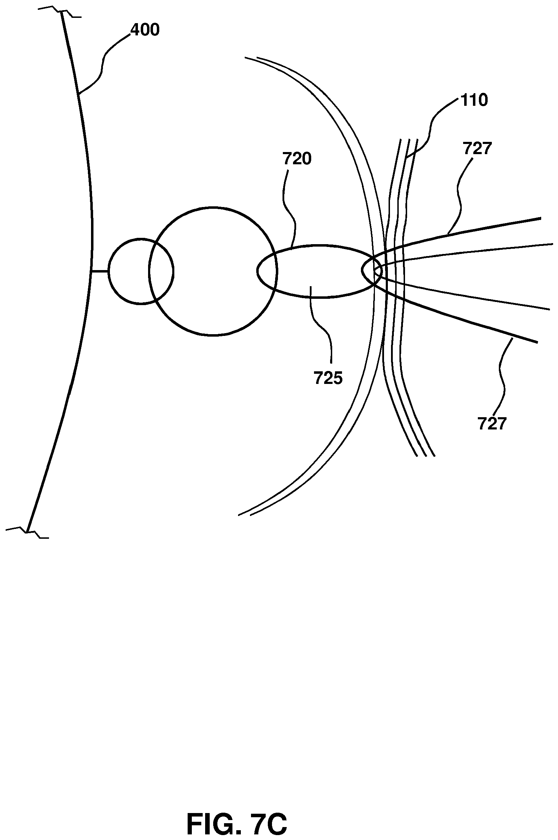

A non-limiting example of such an anchor is provided in **FIG. 7A-7C****.** The anchor **700** facilitates constrained movement of the balloon **400** during use. A fastener **710** may have a first end **712** and a second end **714,** wherein the first end **712** is connected to the balloon and the second end to the anchor mount **720,** either directly **(****FIG. 7A****)** or indirectly via one or more additional anchor elements **(****FIG. 7B-7C****).** Anchor mount **720** may have an opening **725** for receiving pre-sutures, illustrated in this embodiment by two pre-sutures **727,** that may have matching colors to avoid any confusion as to which ends should be tied together to form complete sutures. Anchor mount **720** is illustrated in **FIGs 7A-7B** as ring-shaped. Anchor mount **720** however, can have any of a variety of shapes, particularly for the embodiments where the anchor mount is deformable, such as being formed from suture-threads material that does not substantially degrade in the gastric environment or the gastric wall. For example, **FIG. 7C** illustrates the anchor mount **720** that is deformed into a non-circular, elongated shape.

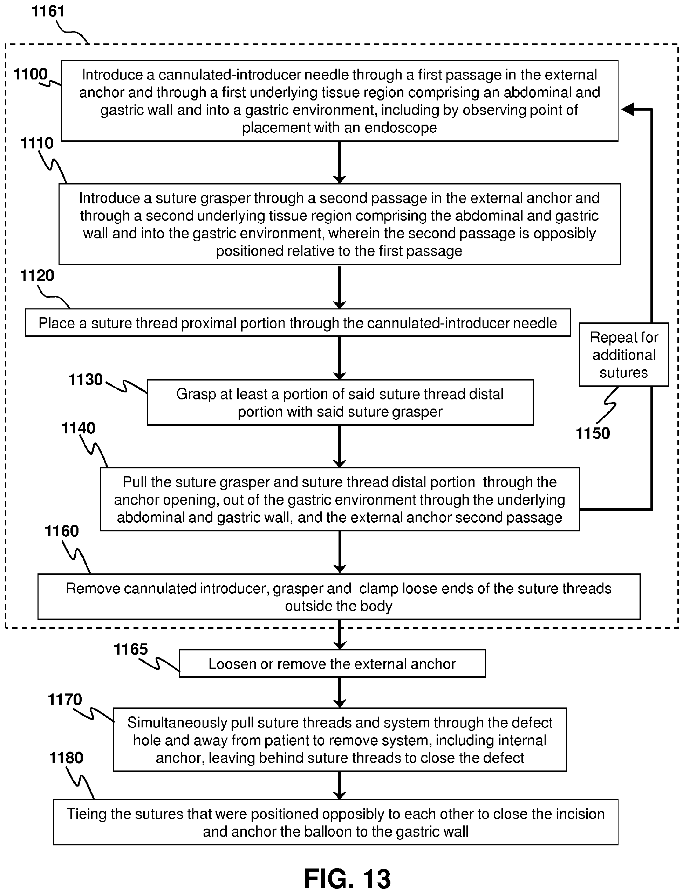

The anchor mount may be connected to the fastener second end, such as via a fastener coupling **730.** The fastener coupling may itself be a ring or other fastener that allows motion of the anchor ring, such as a rotation type motion about one or more axis. As desired, the presutures and gastric cannula **10** may facilitate anchor mount that is at least partially embedded in the gastric wall **(****FIG. 7C****)** and that may extend out from the gastric wall. This anchoring is straightforward, reliable, and automatically achieved as part of the removal of gastric cannula and attendant suturing of the wall defects by presutures **727** (see also **FIGs 13-17****).** The anchor elements, including anchor mount **720** may be formed from a suture thread or material that does not substantially degrade in the gastric environment. In contrast, the presutures **727** may be bioresorbable. Accordingly, if balloon removal is desired, the anchor elements may be simply cut toward the gastric wall, such as via an endoscopic procedure, and the balloon removed from the gastric wall without any need for a surgical intervention. Any remaining ends of non-degradable suture extending from the gastric wall are relatively small and do not adversely impact the gastric wall or environment. Forming the anchor mount from such a non-rigid and shape-changeable material ensures there is an appropriate amount of give in the anchor ends. In an aspect, the anchor may be formed from one or more rings, such as one ring, two rings or three rings.

As provided herein, the anchor can be formed from a single loop or from multiple loops, including two or three loops, so as to provide the appropriate amount of constrained movement and flexibility between the balloon and gastric wall. In the single loop/anchor embodiment, there may be one central or main anchor. In a triple anchor embodiment, there may be one central or main anchor, with two smaller side anchors. The anchor material may be biocompatible, non-dissolvable suture-type material, which allows the anchor to maintain the balloon in the appropriate location without unwanted balloon migration and still allow some slight "give and take" to prevent gastric wall irritation, torsion, etc. Color coded, such as red and blue sutures, either can pass through a main central ring or a side ring, and then the balloon and anchor will close with the same closure method as described for the transabdominal gastric cannula, with optionally reliable embedding of a portion of the anchor mount in the gastric wall and attendant reliable anchoring of the balloon in the gastric or stomach lumen.

Although the anchor has been described in terms of suture materials, the anchors are compatible with any number of additional elements. For example, a magnetic component may be integrated to the anchor rings and suture, to provide an additional means for securing the anchor to the gastric wall.

The anchor is formed of any material, shape, durometer, size and flexibility, as desired, depending on the application of interest and gastric environment.

The rings are easily clipped when it is time to remove the balloon, thus freeing the balloon from the gastric wall without any need for surgical intervention, as the anchors may be accessed endoscopically. While the anchor connection is illustrated as via a suture through a ring or rings, the invention is compatible with many other connection mechanisms and anchors, including but not limited to a fastener, snap device or a clip device.

### Example 4: Closure system removal and anchoring

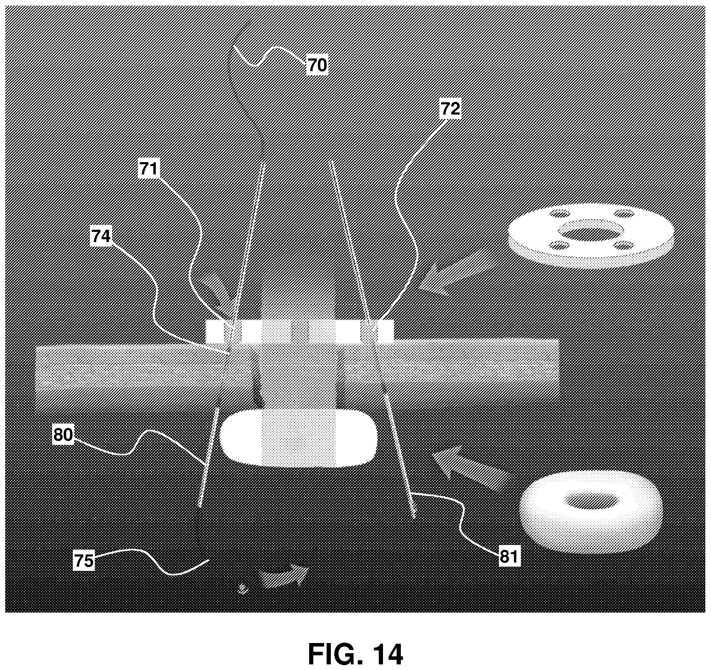

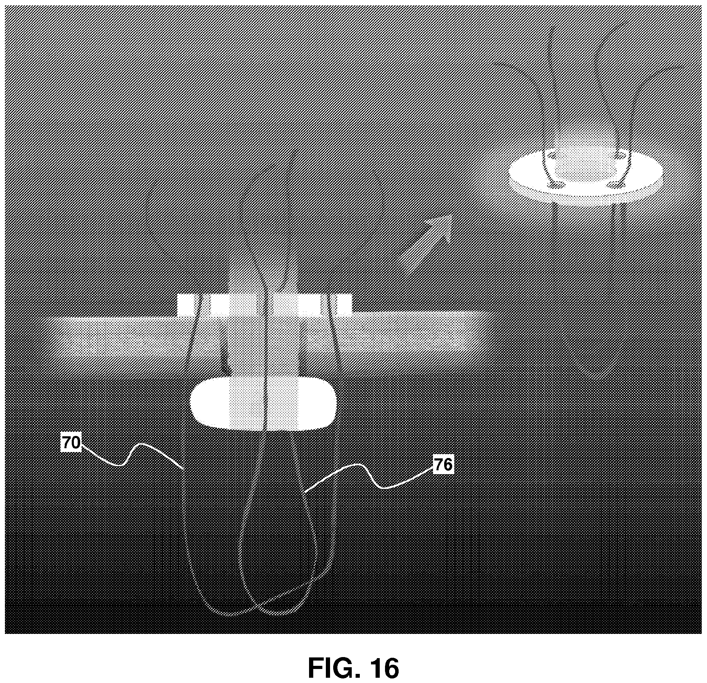

An important benefit of the instant systems and methods is the ability to simply, reliably and robustly close the abdominal incision through which the system traverses, in parallel with balloon anchoring. This is achieved, in part, by the plurality of passages **49** through the flange **48** of the external anchor **40 (****FIG. 2****).** Referring to the flow chart of **FIG. 13** and corresponding diagrams of **FIGs. 14-17****,** passages **49** facilitate guided insertion and removal of one or more suture threads **70 76 (****FIG. 14** and **16****).** In particular, first thread **70** is inserted into first passage **71,** such as by a cannulated-introducer needle **80** containing a suture thread proximal portion **74.** A suture grasper **81** grabs a suture thread distal portion **75** and pulls the suture thread through second passage **72,** that is geometrically opposed to first passage **71.** "Geometrically opposed" in this aspect refers to a pair of passages wherein at least a portion of the external anchor central body **47** is disposed therebetween. Accordingly, with the illustrated configuration, the opposed passages may be 180° opposed (see inset of **FIG. 14**). The invention, however, is compatible with variations on the opposed configuration, so long as a portion of the suture thread traverses the outer-most facing surface of the internal anchor and passes through an anchor mount opening. As desired, a second suture thread **76** is similarly placed through third passage and fourth passage. In this manner, when the system is desired to be removed, the exposed suture threads are pulled away from the patient to remove the system and provide reliably sutures **82** that close the incision outside the abdominal wall and that ensure the anchor and balloon attached thereto is anchored to the gastric wall. As desired, for a plurality of unique suture threads, different color threads are used to ensure the appropriate ends are tied together, such as red-to-red and blue-to-blue.

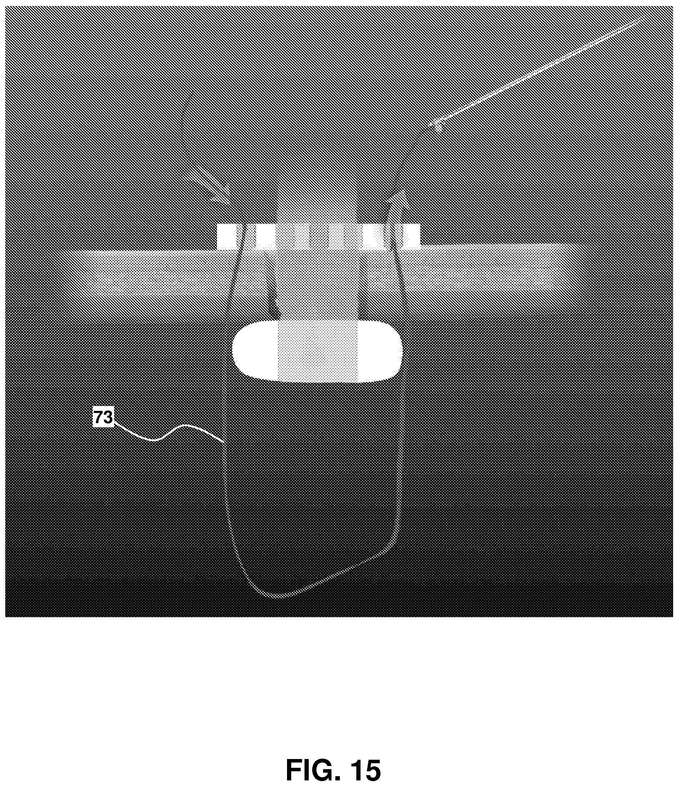

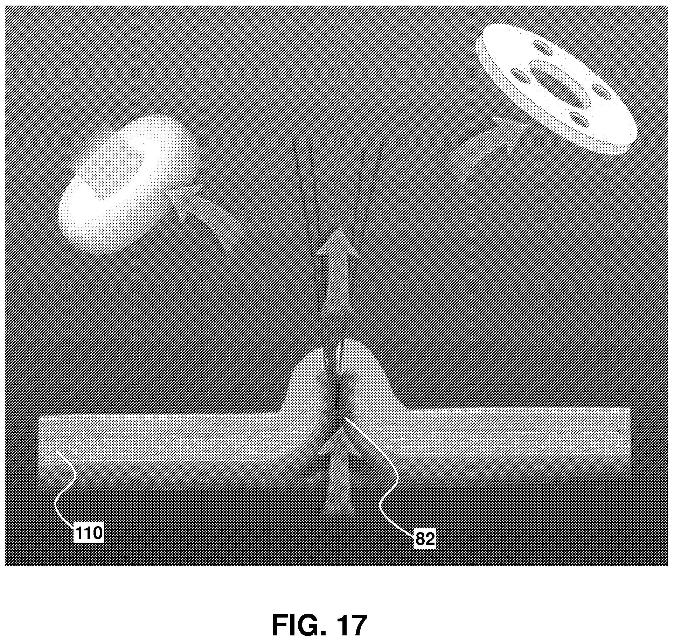

An example of a method for removing the system in a safe and effective manner is summarized in the flow chart of **FIG. 13** and corresponding illustrations **FIG. 14-17****.** Passages **49** in the external anchor facilitate controlled suture positioning at the start of a surgical procedure. In steps **1100** and **1110,** cannulated introducer needle **80** and suture grasper **81** are positioned through first passage **71** and second passage **72 (****FIG. 14****)** and underlying tissue into the gastric space. A suture thread is grasped and pulled through passage **71** and out of passage **72,** thereby providing a threaded loop around the outermost portion of the internal anchor and through an anchor opening (**FIG. 15** and steps **1120 1130** and **1140).** As desired, steps **1100-1140** are repeated to obtain additional suture threads at different orientations from each other (**FIG. 16** and repeating step **1150).** The suture thread ends are pulled away from the patient to remove the system and internal and external anchors, thereby providing sutures **82** outside the abdominal wall **110** and to secure the balloon to the gastric wall to avoid distal migration of the balloon while allowing for constrained balloon movement (**FIG. 17** and steps **1160 1165 1170 1180).** This method is simple and reliable, while minimizing the risk of infection or other complications associated with conventional trocar insertion methods. As illustrated in **FIGs 7A-7C****,** the loose presuture loops may be placed through an opening **725** in the anchor, such as an anchor mount **720,** to facilitate reliable anchoring of a balloon to the gastric wall, including via an at least partially gastric-wall embedded anchor.

### Example 5: System Introduction and Placement

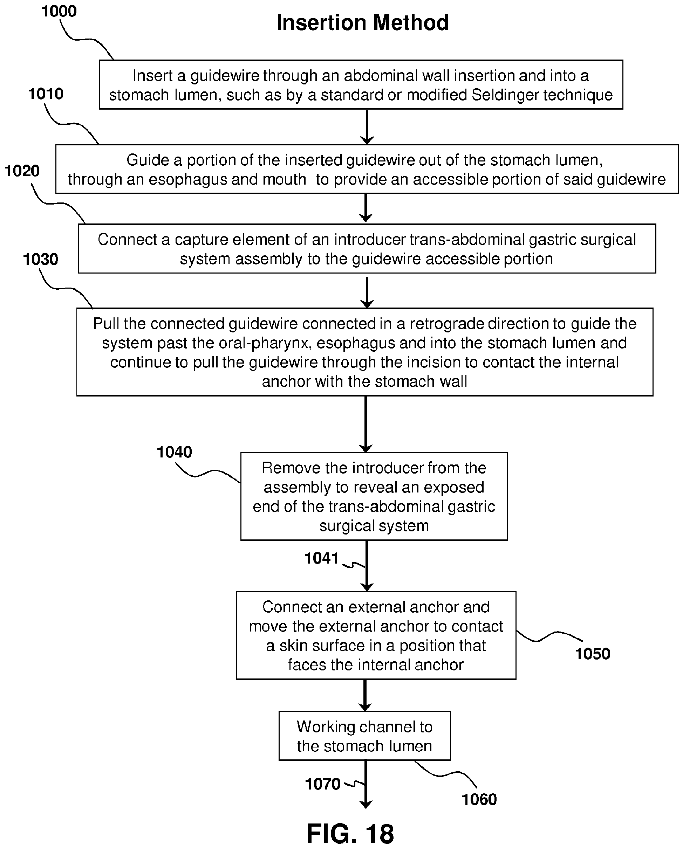

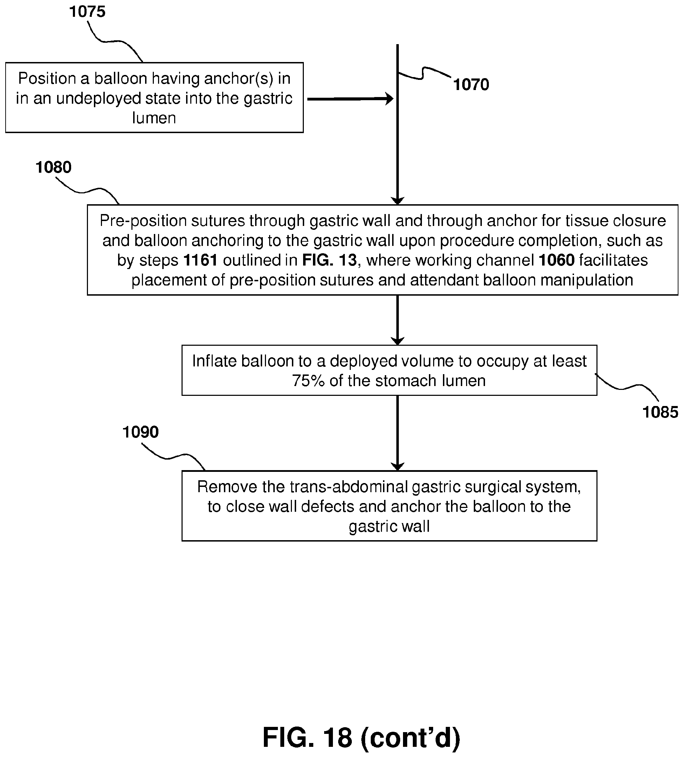

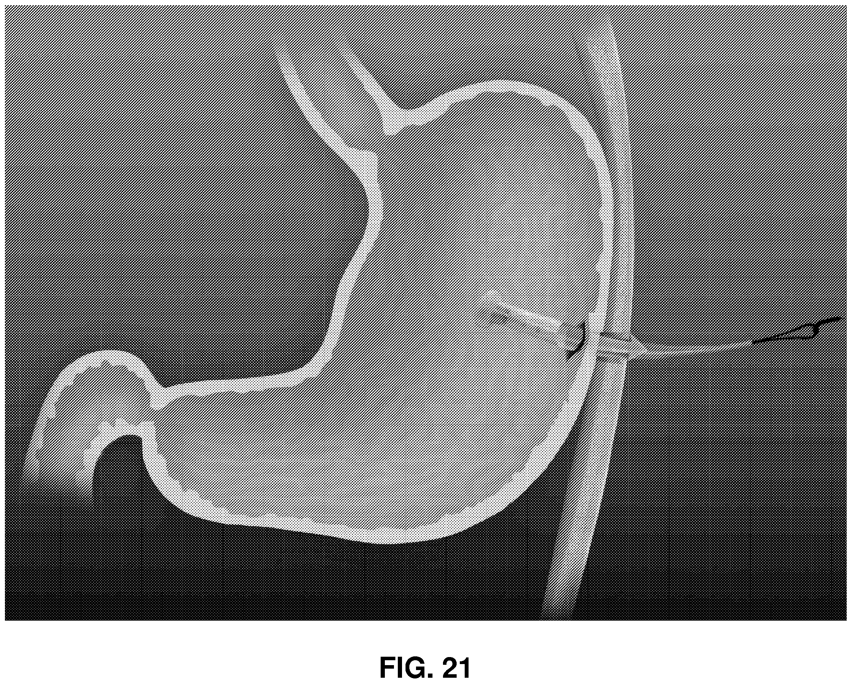

Any one or more of the systems described herein is readily and reliably introduced to a patient. An example of one such method for introducing the system is summarized in the flow chart of **FIG. 18** and corresponding illustrations **FIGs. 19-24**.

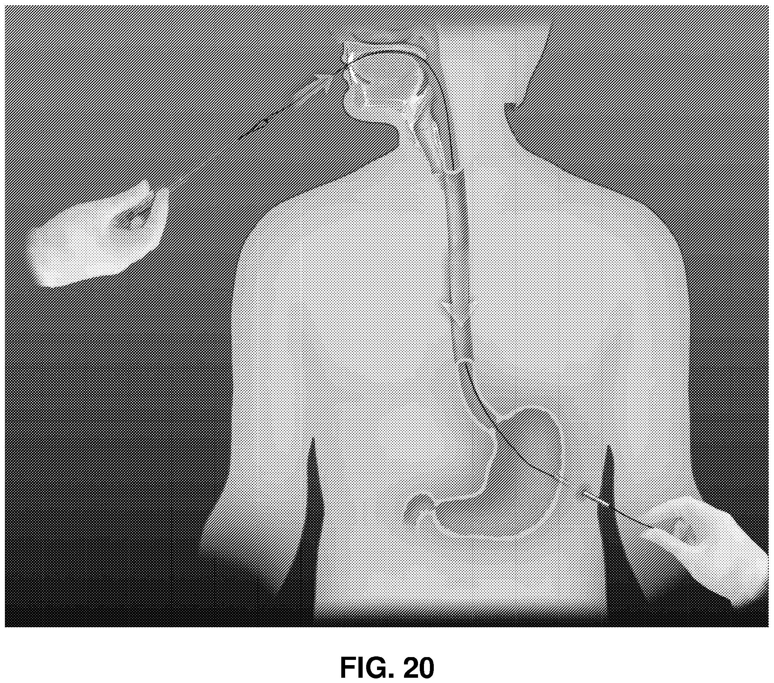

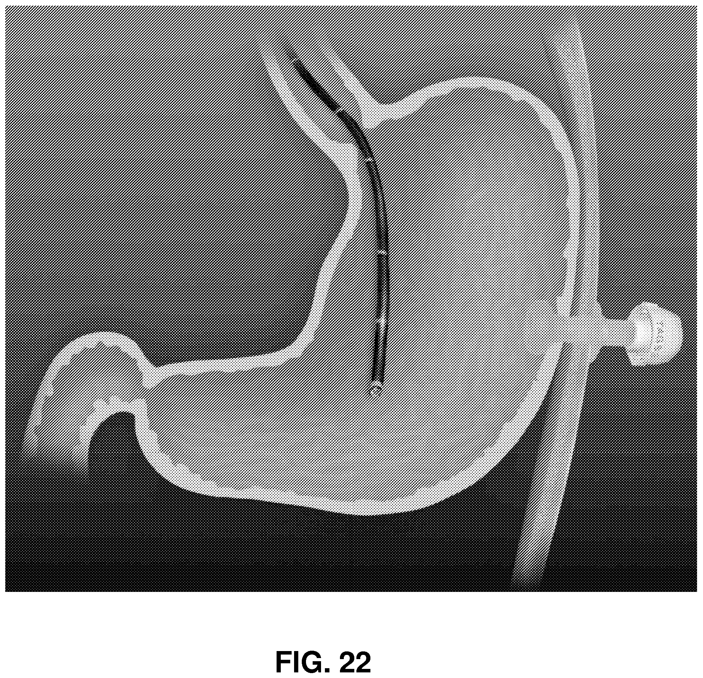

Briefly, in steps **1000, 1010** of **FIG. 18** a guidewire is inserted through an abdominal wall and into the stomach lumen. For a system that will be anchored to the stomach wall, this guidewire may be directly inserted from the abdominal wall and into the stomach lumen. In step **1020,** a capture element of the introducer is connected to the guidewire at one end. At the other end of the introducer the trans-abdominal gastric system is connected thereto **(****FIG. 20****).** The guidewire is then pulled in a retrograde direction, as shown in **FIGs. 20-21** and step **1030** so that the internal anchor contacts the stomach wall **(****FIG. 22****).** In step **1040,** the introducer is removed. The external anchor and cap **(****FIG. 22****)** are connected to the system, so that the system is reliably anchored to the abdominal wall (**FIG. 22** and step **1050**), thereby providing a working channel for subsequent applications, as desired. As desired, a gastric balloon may be introduced to the gastric environment via the working channel or endoscopically **1075.** In addition, a gastric balloon may be manipulated, positioned and anchored with a medical instrument such as a grasper introduced to the gastric lumen via the working channel. As will be appreciated, an equivalent methodology is employed to provide system anchoring to the peritoneal surface to provide extra-luminal access relative to the stomach lumen.

Steps **1075, 1080** and **1085** relate to insertion, positioning and inflation of a gastric balloon for a weight-loss application. In step **1075,** the balloon is inserted into the gastric lumen. This insertion can be by a range of methods, including with the insertion of the cannula summarized in the prior steps, such as a balloon that is integrally packaged with the internal anchor of the transabdominal gastric system, endoscopically either before or after the transabdominal gastric system is inserted, or via the cannula of the transabdominal gastric system.

To facilitate balloon positioning, a medical instrument, such as a laparoscopic grasper, is inserted into the stomach lumen via the gastric cannula working channel **1060.** As desired, additional instruments and working channels may be similarly inserted, to provide device triangulation on various specific points, such as balloon anchors. The balloon is inflated and anchored to the stomach wall at a desired position **1085.** As desired, the system is removed in step **1090,** leaving behind suture-closed wall defects and corresponding wall-anchored inflated balloon, including by an at least partially-embedded anchor.

### STATEMENTS REGARDING VARIATIONS

This application relates to U.S. Pat. App. No. 14/216,666 and PCT App. No. PCT/US2014/030625, each filed Mar. 17, 2014, each of which claims benefit of U.S. Provisional Application Nos. 61/790,709 filed Mar. 15, 2013 and 61/862,463 filed Aug. 5, 2013.

The terms and expressions which have been employed herein are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments, exemplary embodiments and optional features, modification and variation of the concepts herein disclosed may be resorted to by those skilled in the art, and that such modifications and variations are considered to be within the scope of this invention as defined by the appended claims. The specific embodiments provided herein are examples of useful embodiments of the present invention and it will be apparent to one skilled in the art that the present invention may be carried out using a large number of variations of the devices, device components, methods steps set forth in the present description. As will be obvious to one of skill in the art, methods and devices useful for the present methods can include a large number of optional composition and processing elements and steps.

When a group of substituents is disclosed herein, it is understood that all individual members of that group and all subgroups are disclosed separately. When a Markush group or other grouping is used herein, all individual members of the group and all combinations and subcombinations possible of the group are intended to be individually included in the disclosure.

Every formulation or combination of components described or exemplified herein can be used to practice the invention, unless otherwise stated.

Whenever a range is given in the specification, for example, a size range, a time range, or a composition or concentration range, all intermediate ranges and subranges, as well as all individual values included in the ranges given are intended to be included in the disclosure. It will be understood that any subranges or individual values in a range or subrange that are included in the description herein can be excluded from the claims herein.

All patents and publications mentioned in the specification are indicative of the levels of skill of those skilled in the art to which the invention pertains.

As used herein, "comprising" is synonymous with "including," "containing," or "characterized by," and is inclusive or open-ended and does not exclude additional, unrecited elements or method steps. As used herein, "consisting of" excludes any element, step, or ingredient not specified in the claim element. As used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms. The invention illustratively described herein suitably may be practiced in the absence of any element or elements, limitation or limitations which is not specifically disclosed herein.

One of ordinary skill in the art will appreciate that materials, biological materials and methods other than those specifically exemplified can be employed in the practice of the invention without resort to undue experimentation. All art-known functional equivalents, of any such materials and methods are intended to be included in this invention. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. A gastric balloon system for treatment of obesity in a patient comprising:
a transabdominal gastric cannula (10) for gastric balloon insertion or manipulation in a stomach lumen comprising:
an outer end (22);
an inner end (23);
a central portion (24) having an outer-facing surface (25) that extends between said inner end (23) and said outer end (22) and an inner-facing surface (26) that defines a lumen (27);
an internal anchor (30) connected to said inner end (23) and having a surface shape (32) configured to secure the system against an interior surface of a gastric wall;
an external anchor (40) removably and translationally connected to said cannula outer-facing surface and having a surface shape (42) configured to secure said system against a skin surface.
a size-varying gastric balloon (400) configured for delivery to a stomach lumen through the transabdominal gastric cannula (10) or endoscopically, said size-varying gastric balloon (400) comprising:
a gastric balloon surface (410);
an internal volume (402) defined by said gastric balloon surface (410);
a gastric balloon anchor (412) connected to said gastric balloon surface (410) configured to reliably position and anchor said size-varying gastric balloon (400) in a gastric environment;
a fluid conduit (450) that traverses said lumen (27) of the transabdominal gastric cannula (10), the fluid conduit (450) having a first fluid end (451) and a second fluid end (452):
wherein said first fluid end (451) is connected to said size-varying gastric balloon (400) and in fluid communication with said internal volume (402) and said second fluid end (452) is positioned external to the size-varying gastric balloon (400) internal volume (402);
wherein said fluid conduit (450) is configured to introduce a fluid (460) to said internal volume (402) to increase said internal volume (402) to a deployed internal volume and provide a gastric balloon deployed configuration;
said size-varying gastric balloon (400) having an annulus (430) in said deployed configuration configured to align in the stomach to pass food and liquid in a direction from an esophageal exit toward a pyloric sphincter.

2. The gastric balloon system of claim 1, wherein said gastric balloon anchor (412) is connected to said transabdominal gastric cannula (10), thereby tethering said gastric balloon in a desired position within the stomach lumen.

3. The gastric balloon system of claim 1, wherein said fluid conduit (450) is connected to an anterior portion of said size-varying gastric balloon (400).

4. The gastric balloon system of claim 1, further comprising a second fluid conduit and a second transabdominal gastric cannula through which each fluid conduit independently traverses, each first fluid conduit end connected to an anterior portion of said size-varying gastric balloon (400) with the connections separated from each other by a fluid conduit separation distance that is greater than or equal to 15 cm to provide at least two ports for inflation, deflation, or inflation and deflation.

5. The gastric balloon system of claim 1, wherein said transabdominal gastric cannula (10) has a low profile, and after deployment to said gastric balloon deployed configuration, said transabdominal gastric cannula (10) is retained in the patient wherein said fluid conduit (450) is configured to remove a fluid from said internal volume (402) to decrease said internal volume (402).

6. The gastric balloon system of claim 1, wherein said fluid conduit (450) is connected to a fluid source external to the patient for introducing a fluid to said internal volume (402) to control said size-varying gastric balloon (400) shape and size, wherein said fluid is a gas, a liquid, or a gel.

7. The gastric balloon system of claim 1, wherein said fluid conduit (450) is connected to said transabdominal gastric cannula (10).

8. The gastric balloon system of claim 1, wherein said annulus (430) has an average diameter that is greater than or equal to 0.5 cm and less than or equal to 5 cm and the size-varying gastric balloon (400) has a deployed volume that occupies at least 75% of the stomach lumen.

9. The gastric balloon system of claim 8, wherein said annulus (430) has a length that is greater than or equal to 5 cm and less than or equal to 50 cm and said annulus (430) is curved and during use said annulus (430) has:
an entry end configured to be aligned and separated from a patient's esophageal exit; and
an exit end configured to be aligned and separated from a patient's pyloric sphincter.

10. The gastric balloon system of claim 1, wherein during use:
at least 75% of the stomach lumen is occupied by a deployed balloon and less than 25% of the stomach lumen is unoccupied by the deployed balloon, wherein the gastric balloon anchor (412) mechanically connects the deployed balloon to a stomach surface to maintain a deployed balloon position relative to the gastric lumen during long-term use.

11. The gastric balloon system of claim 10, wherein during use an entry end and an exit end are substantially fixably positioned within a gastric environment by one or more gastric balloon anchors (412) that substantially constrain gastric balloon migration.

12. The gastric balloon system of claim 11, wherein said gastric balloon anchor (412) comprises a first end operably connected to said gastric balloon surface (410) and a second end configured to connect to a suture extending from a gastric wall during use.

13. The gastric balloon system of claim 1, wherein said gastric balloon anchor (412) comprises said fluid conduit (450) connected to said internal volume (402), wherein said fluid conduit (450) comprises a percutaneous catheter that passes through and is connected to said transabdominal gastric cannula (10).

14. The gastric balloon system of claim 1, further comprising an elongated sleeve configured for insertion into a portion of a patient's small intestine, said elongated sleeve having a lumen and a proximal end connected thereto, said proximal end configured for positioning at or adjacent to a pyloric sphincter and aligned with said gastric balloon annulus (430) during use.

15. The gastric balloon system of any of claims 1-14, wherein the gastric balloon anchor (412) comprises:
an anchor mount having an opening for receiving one or more pre-sutures to at least partially embed the anchor mount in the gastric wall;
a fastener having:
a first end configured to connect to a gastric balloon surface (410); and
a second end configured to connect to the anchor mount.

## Patentansprüche

1. Ein Magenballonsystem zur Behandlung von Fettleibigkeit in einem Patienten, umfassend:
eine transabdominale Magenkanüle (10) zur Einführung oder Manipulation eines Magenballons in einem Magenlumen, umfassend:
ein äußeres Ende (22);
ein inneres Ende (23);
ein Mittelteil (24) mit einer nach außen gerichteten Oberfläche (25), die sich zwischen dem inneren Ende (23) und dem äußeren Ende (22) erstreckt, und
einer nach innen gerichteten Oberfläche (26), die einen Hohlraum (27) bildet;
eine mit dem inneren Ende (23) verbundene interne Verankerung (30) mit einer Oberflächenform (32), die ausgebildet ist, das System gegen eine innere Oberfläche einer Magenwand zu sichern;
eine lösbar und verschiebbar mit der nach außen gerichteten Oberfläche der Kanüle verbundene externe Verankerung (40) mit einer Oberflächenform (42), die ausgebildet ist, das System gegen eine Hautoberfläche zu sichern,
ein größenveränderlicher Magenballon (400), der für die Zuführung in ein Magenlumen durch die transabdominale Magenkanüle (10) oder endoskopisch ausgebildet ist, wobei der größenveränderliche Magenballon (400) umfasst:
eine Magenballonoberfläche (410);
ein internes Volumen (402), das durch die Magenballonoberfläche (410) ausgebildet ist;
eine mit der Magenballonoberfläche (410) verbundene Magenballonverankerung (412), die ausgebildet ist, um den größenveränderliche Magenballon (400) in einer Magenumgebung zuverlässig
zu positionieren und zu verankern;
eine Fluidleitung (450), die den Hohlraum (27) der transabdominalen Magenkanüle (10) durchquert, wobei die Fluidleitung (450) ein erstes Fluidende (451) und ein zweites Fluidende (452) aufweist:
wobei das erste Fluidende (451) mit dem größenveränderlichen Magenballon (400) verbunden ist und in fluidischer Verbindung mit dem internen Volumen (402) steht und das zweite Fluidende (452) ist extern zu dem internen Volumen (402) des größenveränderlichen Magenballons (400) positioniert;
wobei die Fluidleitung (450) ausgebildet ist, ein Fluid (460) in das interne Volumen (402) einzuführen, um das interne Volumen (402) zu einem internen Einsatzvolumen zu vergrößern und eine Einsatzkonfiguration des Magenballons bereitzustell en;
der größenveränderliche Magenballon (400) weist in der Einsatzkonfiguration einen Ringraum (430) auf, der ausgebildet ist, sich im Magen auszurichten, um Nahrung und Flüssigkeit in einer Richtung von einem Speiseröhrenausgang zu einem Magenpförtnermuskel zu übergeben.

2. Das Magenballonsystem nach Anspruch 1, wobei die Magenballonverankerung (412) mit der transabdominalen Magenkanüle (10) verbunden ist, wodurch der Magenballon in einer gewünschten Position innerhalb des Magenlumens angebunden wird.

3. Das Magenballonsystem nach Anspruch 1, wobei die Fluidleitung (450) mit einem vorderen Teil des größenveränderlichen Magenballons (400) verbunden ist.

4. Das Magenballonsystem nach Anspruch 1, weiter umfassend eine zweite Fluidleitung und eine zweite transabdominale Magenkanüle, die jede Fluidleitung unabhängig durchquert, jedes erste Fluidende ist mit einem vorderen Teil des größenveränderlichen Magenballons (400) verbunden, wobei die Verbindungen durch einen Fluidleitungstrennungsabstand, der größer oder gleich 15 cm ist, voneinander getrennt sind, um mindestens zwei Anschlüsse für Inflation, Deflation oder Inflation und Deflation bereitzustellen.

5. Das Magenballonsystem nach Anspruch 1, wobei die transabdominale Magenkanüle (10) ein niedriges Profil aufweist, und nach dem Bereitstellen an die Einsatzkonfiguration des Magenballons, wird die transabdominale Magenkanüle (10) in dem Patienten einbehalten, wobei die Fluidleitung (450) ausgebildet ist, ein Fluid aus dem internen Volumen (402) zu entfernen, um das interne Volumen (402) zu verringern.

6. Das Magenballonsystem nach Anspruch 1, wobei die Fluidleitung (450) mit einer Patienten-externen Fluidquelle verbunden ist, um ein Fluid in das interne Volumen (402) einzuführen, um die Form und Größe des größenveränderlichen Magenballons (400) zu steuern, wobei das Fluid ein Gas, eine Flüssigkeit oder ein Gel ist.

7. Das Magenballonsystem nach Anspruch 1, wobei die Fluidleitung (450) mit der transabdominalen Magenkanüle (10) verbunden ist.

8. Das Magenballonsystem nach Anspruch 1, wobei der Ringraum (430) einen durchschnittlichen Durchmesser aufweist, der größer oder gleich 0.5 cm und kleiner oder gleich 5 cm ist, und der größenveränderliche Magenballon (400) ein Einsatzvolumen aufweist, dass mindestens 75% des Magenlumens belegt.

9. Das Magenballonsystem nach Anspruch 8, wobei der Ringraum (430) eine länge aufweist, die größer oder gleich 5cm und kleiner oder gleich 50 cm ist, und der Ringraum (430) gebogen ist und der Ringraum (430) während der Verwendung aufweist:
ein Eingangsende, das ausgebildet ist, ausgerichtet und von einem Speiseröhrenausgang eines Patienten getrennt zu werden; und
ein Ausgangsende, das ausgebildet ist, ausgerichtet und von einem Magenpförtnermuskel eines Patienten getrennt zu werden.

10. Das Magenballonsystem nach Anspruch 1, wobei während der Verwendung:
mindestens 75% des Magenlumens durch einen eingesetzten Ballon belegt sind und weniger als 25% des Magenlumens durch den eingesetzten Ballon unbelegt sind, wobei die Magenballonverankerung (412) den eingesetzten Ballon mechanisch mit einer Magenoberfläche verbindet, um eine Position des eingesetzten Ballons relativ zu dem Magenlumen während einer Langzeitverwendung zu erhalten.

11. Das Magenballonsystem nach Anspruch 10, wobei während der Verwendung ein Eingangsende und ein Ausgangsende innerhalb einer Magenumgebung durch eine oder mehrere Magenballonverankerungen (412), die eine Magenballonwanderung wesentlich einschränken, im Wesentlichen fixierbar positioniert sind.

12. Das Magenballonsystem nach Anspruch 11, wobei die Magenballonverankerung (412) ein erstes Ende, das operativ mit der Magenballonoberfläche (410) verbunden ist, und ein zweites Ende, das ausgebildet ist, mit einem Nahtfaden, der sich während der Verwendung aus einer Magenwand erstreckt, verbunden zu werden, umfasst.

13. Das Magenballonsystem nach Anspruch 1, wobei die Magenballonverankerung (412) die mit dem internen Volumen (402) verbundene Fluidleitung (450) umfasst, wobei die Fluidleitung (450) einen perkutanen Katheter umfasst, der die transabdominale Magenkanüle (10) durchquert und mit dieser verbunden ist.

14. Das Magenballonsystem nach Anspruch 1, weiter umfassend eine längliche Hülse, die ausgebildet ist, um in einen Teil eines Dünndarms eines Patienten eingeführt zu werden, wobei die längliche Hülse einen Hohlraum und ein damit verbundenes proximales Ende aufweist, wobei das proximale Ende ausgebildet ist, um während der Verwendung an oder neben einem Magenpförtnermuskel positioniert und mit dem Magenballonringraum (430) ausgerichtet zu werden.

15. Das Magenballonsystem nach einem der Ansprüche 1 - 14, wobei die Magenballonverankerung (412) umfasst:
eine Verankerungshalterung, die eine Öffnung zum Aufnehmen eines oder
mehrerer Prä-Nahtfäden, um die Verankerungshalterung zumindest teilweise in der Magenwand einzubinden;
ein Befestigungsmittel aufweisend:
ein erstes Ende, ausgebildet, um mit einer Magenballonoberfläche (410) verbunden zu werden; und
ein zweites Ende, ausgebildet, um mit der Verankerungshalterung verbunden zu werden.

## Revendications

1. Système de ballonnet gastrique pour le traitement de l'obésité chez un patient, comprenant :
une canule gastrique transabdominale (10) pour l'insertion ou la manipulation d'un ballonnet gastrique dans une lumière de l'estomac, comprenant :
une extrémité externe (22) ;
une extrémité interne (23) ;
une partie centrale (24) ayant une surface orientée vers l'extérieur (25) qui s'étend entre ladite extrémité interne (23) et ladite extrémité externe (22) et une surface orientée vers l'intérieur (26) qui définit une lumière (27) ;
un ancrage interne (30) relié à ladite extrémité interne (23) et ayant une forme de surface (32) configurée pour fixer le système contre une surface intérieure d'une paroi gastrique ;
un ancrage externe (40) relié de manière amovible et à translation à ladite surface de canule orientée vers l'extérieur et ayant une forme de surface (42) configurée pour fixer ledit système contre une surface de peau ;
un ballonnet gastrique à taille variable (400) configuré pour une pose dans une lumière de l'estomac par l'intermédiaire de la canule gastrique transabdominale (10) ou par voie endoscopique, ledit ballonnet gastrique à taille variable (400) comprenant :
une surface de ballonnet gastrique (410) ;
un volume interne (402) défini par ladite surface de ballonnet gastrique (410) ;
un ancrage de ballonnet gastrique (412) relié à ladite surface de ballonnet gastrique (410), configuré pour positionner et ancrer de manière fiable ledit ballonnet gastrique à taille variable (400) dans un environnement gastrique ;
un conduit de fluide (450) qui traverse ladite lumière (27) de la canule gastrique transabdominale (10), le conduit de fluide (450) ayant une première extrémité de fluide (451) et une seconde extrémité de fluide (452) ;
ladite première extrémité de fluide (451) étant reliée audit ballonnet gastrique à taille variable (400) et en communication fluidique avec ledit volume interne (402), et ladite seconde extrémité de fluide (452) étant positionnée à l'extérieur du volume interne (402) du ballonnet gastrique à taille variable (400) ;
ledit conduit de fluide (450) étant configuré pour introduire un fluide (460) dans ledit volume interne (402) pour accroître ledit volume interne (402) jusqu'à un volume interne déployé et obtenir une configuration déployée de ballonnet gastrique ;
ledit ballonnet gastrique à taille variable (400) ayant un espace annulaire (430) dans ladite configuration déployée, configuré pour s'aligner dans l'estomac pour faire passer des aliments et des liquides dans une direction allant d'une sortie de l'oesophage vers un sphincter du pylore.

2. Système de ballonnet gastrique selon la revendication 1, dans lequel ledit ancrage de ballonnet gastrique (412) est relié à ladite canule gastrique transabdominale (10), attachant ainsi ledit ballonnet gastrique dans une position souhaitée à l'intérieur de la lumière de l'estomac.

3. Système de ballonnet gastrique selon la revendication 1, dans lequel ledit conduit de fluide (450) est relié à une partie antérieure dudit ballonnet gastrique à taille variable (400).

4. Système de ballonnet gastrique selon la revendication 1, comprenant en outre un second conduit de fluide et une seconde canule gastrique transabdominale que chaque conduit de fluide traverse de manière indépendante, chaque première extrémité de conduit de fluide étant reliée à une partie antérieure dudit ballonnet gastrique à taille variable (400) avec les raccordements séparés l'un de l'autre d'une distance de séparation de conduit de fluide qui est supérieure ou égale à 15 cm pour fournir au moins deux orifices pour le gonflage, le dégonflage, ou le gonflage et dégonflage.

5. Système de ballonnet gastrique selon la revendication 1, dans lequel ladite canule gastrique transabdominale (10) a un profil bas et, après déploiement dans ladite configuration déployée de ballonnet gastrique, ladite canule gastrique transabdominale (10) est retenue dans le patient, ledit conduit de fluide (450) étant configuré pour retirer un fluide dudit volume interne (402) pour réduire ledit volume interne (402).

6. Système de ballonnet gastrique selon la revendication 1, dans lequel ledit conduit de fluide (450) est relié à une source de fluide à l'extérieur du patient pour introduire un fluide dans ledit volume interne (402) pour contrôler la forme et la taille dudit ballonnet gastrique à taille variable (400), ledit fluide étant un gaz, un liquide ou un gel.

7. Système de ballonnet gastrique selon la revendication 1, dans lequel ledit conduit de fluide (450) est relié à ladite canule gastrique transabdominale (10).

8. Système de ballonnet gastrique selon la revendication 1, dans lequel ledit espace annulaire (430) a un diamètre moyen qui est supérieur ou égal à 0,5 cm et inférieur ou égal à 5 cm, et le ballonnet gastrique à taille variable (400) a un volume déployé qui occupe au moins 75 % de la lumière de l'estomac.

9. Système de ballonnet gastrique selon la revendication 8, dans lequel ledit espace annulaire (430) a une longueur qui est supérieure ou égale à 5 cm et inférieure ou égale à 50 cm et ledit espace annulaire (430) est incurvé et, en utilisation, ledit espace annulaire (430) a :
une extrémité d'entrée configurée pour être alignée avec et séparée de la sortie de l'œsophage d'un patient ; et
une extrémité de sortie configurée pour être alignée avec et séparée du sphincter du pylore d'un patient.

10. Système de ballonnet gastrique selon la revendication 1, dans lequel, en utilisation :
au moins 75 % de la lumière de l'estomac est occupé par un ballonnet déployé et moins de 25 % de la lumière de l'estomac n'est pas occupée par le ballonnet déployé, l'ancrage de ballonnet gastrique (412) reliant mécaniquement le ballonnet déployé à une surface de l'estomac pour maintenir une position de ballonnet déployé par rapport à la lumière gastrique pendant une utilisation à long terme.

11. Système de ballonnet gastrique selon la revendication 10, dans lequel, en utilisation, une extrémité d'entrée et une extrémité de sortie sont positionnées de manière sensiblement fixe à l'intérieur d'un environnement gastrique par un ou plusieurs ancrages de ballonnet gastrique (412) qui limitent sensiblement une migration du ballonnet gastrique.

12. Système de ballonnet gastrique selon la revendication 11, dans lequel ledit ancrage de ballonnet gastrique (412) comprend une première extrémité reliée de manière fonctionnelle à ladite surface de ballonnet gastrique (410) et une seconde extrémité configurée pour être reliée à une suture s'étendant à partir d'une paroi gastrique pendant l'utilisation.

13. Système de ballonnet gastrique selon la revendication 1, dans lequel ledit ancrage de ballonnet gastrique (412) comprend ledit conduit de fluide (450) relié audit volume interne (402), ledit conduit de fluide (450) comprenant un cathéter percutané qui traverse et est relié à ladite canule gastrique transabdominale (10).

14. Système de ballonnet gastrique selon la revendication 1, comprenant en outre un manchon allongé configuré pour une insertion dans une partie de l'intestin grêle d'un patient, ledit manchon allongé ayant une lumière et une extrémité proximale reliée à celle-ci, ladite extrémité proximale étant configurée pour être positionnée à ou adjacente à un sphincter du pylore et alignée avec ledit espace annulaire de ballonnet gastrique (430) pendant l'utilisation.

15. Système de ballonnet gastrique selon l'une quelconque des revendications 1 à 14, dans lequel l'ancrage de ballonnet gastrique (412) comprend :
une monture d'ancrage ayant une ouverture pour recevoir une ou plusieurs présutures pour au moins partiellement intégrer la monture d'ancrage dans la paroi gastrique ;
un élément de fixation ayant :
une première extrémité configurée pour être reliée à une surface de ballonnet gastrique (410) ; et
une seconde extrémité configurée pour être reliée à la monture d'ancrage.
